# EUROPEAN PATENT SPECIFICATION

(11) **EP 3 833 741 B1**
(45) Date of publication and mention of the grant of the patent: **15.11.2023**
(21) Application number: 19769576.0
(22) Date of filing: 05.08.2019
(51) Int. Cl.: G01N 33/50, A61L 27/38, A61L 27/50, C12N 5/071

(54) **MODEL FOR IN-VITRO SIMULATION OF THE BEHAVIOUR OF DYSFUNCTIONAL VESSELS**
MODELL ZUR IN-VITRO-SIMULATION DES VERHALTENS VON DYSFUNKTIONELLEN GEFÄßEN
MODÈLE DE SIMULATION IN VITRO DU COMPORTEMENT DE VAISSEAUX DYSFONCTIONNELS

(30) Priority: 07.08.2018 IT 201800007946
(43) Date of publication of application: 16.06.2021
(73) Proprietor: 1Lab SA, 6928 Manno (CH)
(72) Inventor: PERFLER, Enrico, 6928 Manno (CH); BIFFI, Andrea, 6928 Manno (CH); FARINA, Stefano, 6928 Manno (CH)
(74) Representative: Benedetto, Marco
(86) International application number: PCT/IB2019/056652
(87) International publication number: WO 2020/031067

(56) References cited:
- WO-A1-2012/175797
- WO-A1-2015/061907
- WO-A1-2018/053265
- WO-A1-2018/063099
- WO-A1-2019/034966
- WO-A2-03/082145
- US-A1- 2004 037 813
- US-A1- 2007 243 574
- Robert J Fisher ET AL: "Controlling Tissue Microenvironments: Biomimetics, Transport Phenomena, and Reacting Systems" In: "Tissue Engineering II", 1 January 2007 (2007-01-01), Springer, XP055574046, ISBN: 978-3-540-36186-2 pages 1-73, page 40, paragraph 3.5.2 - page 4.5, paragraph 3.5.3
- TAKEHISA MATSUDA: "Fabrication Factory for Tubular Vascular Tissue Mimics based on Automated Rolling Manipulation and Thermo-Responsive Polymers", JOURNAL OF TISSUE SCIENCE & ENGINEERING, vol. 05, no. 01, 1 January 2014 (2014-01-01), page 1, XP055574055, DOI: 10.4172/2157-7552.1000134
- ZHANG X ET AL: "Dynamic culture conditions to generate silk-based tissue-engineered vascular grafts", BIOMATERIALS, ELSEVIER SCIENCE PUBLISHERS BV., BARKING, GB, vol. 30, no. 19, 1 July 2009 (2009-07-01), pages 3213-3223, XP026130537, ISSN: 0142-9612, DOI: 10.1016/J.BIOMATERIALS.2009.02.002 [retrieved on 2009-02-20]
- LIU YUANYUAN ET AL: "Fabrication of triple-layered bifurcated vascular scaffold with a certain degree of three-dimensional structure", AIP ADVANCES, AMERICAN INSTITUTE OF PHYSICS, 2 HUNTINGTON QUADRANGLE, MELVILLE, NY 11747, vol. 8, no. 1, 9 January 2018 (2018-01-09) , XP012225292, DOI: 10.1063/1.5015947 [retrieved on 2018-01-09]
- PETER S. MCFETRIDGE ET AL: "Endothelial and Smooth Muscle Cell Seeding onto Processed Ex Vivo Arterial Scaffolds Using 3D Vascular Bioreactors", ASAIO JOURNAL, vol. 50, no. 6, 1 November 2004 (2004-11-01), pages 591-600, XP055181112, ISSN: 1058-2916, DOI: 10.1097/01.MAT.0000144365.22025.9B
- SEBASTIAN SCHUERLEIN ET AL: "A versatile modular bioreactor platform for Tissue Engineering", BIOTECHNOLOGY JOURNAL, vol. 12, no. 2, 1 February 2017 (2017-02-01), page 1600326, XP055521499, DE ISSN: 1860-6768, DOI: 10.1002/biot.201600326

## Description

The present invention refers to a model for in-vitro simulation of the behaviour of dysfunctional human vessels, such as for example vessels affected by aneurysm, stenosis or sclerosis plaques, as an instrument for testing medical devices and drugs with the aim of verifying the effectiveness and safety thereof prior to use thereof on humans. Specifically, the present invention refers to an in vitro model of a vascular structure, preferably a substantially tubular-shaped synthetic vascular structure having dysfunctional anatomical and physiological characteristics, simulating the vascular structure of a healthy subject whose vascular structure has been damaged or deformed or deteriorated due to a damage selected from among the group comprising or, alternatively, consisting of aneurysm, stenosis, sclerosis plaques, forms of tumours or cardiomyopathies having the characteristics as claimed in the attached claims. Furthermore, the present invention also refers to a reliable and reproducible industrialisation process for eliminating air bubbles for producing an engineered vascular tissue for the in vitro test of medicinal products for human use and veterinarian products for animal use.

It is known that the development of a medicinal product entails a long and sensitive process. Basically, in the medicinal or veterinarian product development process, whether a drug or medical device, prior to using the product in humans or in animals it is important to be able to determine the type of effects on the tissue/s with which it comes into contact so as to evaluate the aspects relating both to biological safety and to the efficiency of the medicinal product and foretell potential problems relating to the use thereof.

In this context, the evaluation of the biological safety and efficiency of the medicinal product is extensive and complex while the evaluation of the interaction with the tissue/s of only one constituent material cannot be considered as isolated from the overall planning of the medicinal or veterinarian product which must be evaluated as a whole and in a context that can reproduce the conditions of use as faithfully as possible.

Today, the evaluation of biological safety and of the efficiency of a medicinal or veterinarian product are based on in vitro , ex vivo tests and on animal models that have significant differences with respect to the final conditions of use. In particular, the animal models have significant physiological differences that complicate the transposition of the validity of the results to humans. Such differences are observable even further when evaluating medicinal products for human use or veterinarian products for animal use that provide for the use thereof in the cardiovascular and peripheral vascular region, such as, for example, heart valves, stents, grafts, catheters, bandages and nets.

Unfortunately, the evaluation of the interaction of medicinal products for human use or veterinarian products for animal use with the vascular tissues and with the blood so as to be able to establish the biological safety and efficiency is crucial and the models currently in use reveal major limits and drawbacks both anatomical/structural and regarding the blood composition.

Furthermore, tests on animals have been at the centre of an intense and controversial debate around the issue whether using animals for biomedical testing can be considered morally acceptable. This issue led to the 3R principle already back in 1959, and it still remains a hotly disputed current topic in debates and in the international research programmes. The 3R principle refers to three key concepts: replacement, reduction and refinement. The 3R principle argues that research in the biomedical industry should aim at, with utmost effort possible, replacing or substituting the animal model with an alternative model; reducing the number of animals used in a given experimental protocol as much as possible; refining, i.e. improving the experimental conditions to which the animals are subjected.

It is known in the art in the context of reconstruction of tissues ex vivo that is crucial to characterize and manipulate the cellular microenvironment for a successful implementation of cell-based bioengineered systems and how is essential the knowledge of fundamental biomimetics, transport phenomena, and reaction engineering concepts for the design and development of tissue engineering systems such as syntetic or terapeutic products ("Controlling Tissue Microenvironments: Biomimetics, Transport Phenomena and Reacting Systems", Robert J Fisher at al.).

The vascular tissue engineering industry shows that it is crucial to be able to produce a continuous (i.e. having a monolayer of confluent cells) and functional endothelium mainly consisting of endothelial cells (ECs).

It is known in the art a methodology for fabrication of tubular vascular tissue using thermoresponsive polymers which have temperature-induced reversible phase transition characteristics ("Fabrication Factory for Tubular Vascular Tissue Mimics based on automated Rolling Manipulation and Thermo-Responsive Polymers", Takehisa Matsuda et al)

It is also known in the art, a tissue-engineered construct that mimicked the structure of blood vessels using tubular electrospun silk fibroin scaffolds with suitable mechanical properties. Human coronary artery smooth muscle cells and human aortic endothelial cells (HAECs) were sequentially seeded onto the luminal surface of the tubular scaffolds and cultivated under physiological pulsatile flow ("Dynamic culture conditions to generate silk-based tissue-engineered vascular grafts", Zhang X et al).

A three-layered bifurcated vascular scaffold is presented with a curved structure using a technique which combines 3D printed molds and casting hydrogel and ink to create vessel-mimicking constructs with customizable structural parameters, providing, therefore, a feasible approach for vascular scaffold construction. Enzymatically-crosslinked gelatin was used as the scaffold material. On the scaffold human umbilical cord derived endothelial cells (HUVECs) were seeded on the scaffolds and cultured ("Fabrication of triple-layered bifurcated vascular scaffold with a certain degree of three-dimensional structure" Liu Y et al).

The production of continuous and functional endothelium is a crucial factor towards promoting scaffold endothelisation and guaranteeing appropriate efficiency of the tissue-engineered constructs (consisting of scaffolds and cells), including preventing thrombosis and stenosis once said constructs have been implanted. US2004/037813 describes the formation and use of electro processed collagen, including use as an extracellular matrix and its use in forming engineered tissue.

It is also known in the art the investigation on the seeding efficiency and retention to an acellular ex vivo scaffold of endothelial cells and vascular smooth muscle cells under simulated in vivo shear and flow conditions ("Endothelial and Smooth Muscle Cell Seeding onto Processed Ex Vivo Arterial Scaffolds Using 3D Vascular Bioreactors", McFetridge at al).

The in vitro generation of vascular endothelium or engineered vascular construct/tissue, in short provides for the following steps:
(1) seeding endothelial cells in the lumen of the scaffold and ensuing adhesion thereof,
(2) cell growth/proliferation and organisation as a function of mechanical stimuli (such as for example the flow of a fluid) to which they are subjected, and
(3) generating one or more layers of functional endothelial cells.

Due to the crucial importance of the seeding step, many research groups at university level have invested in research over the last decades creating various techniques for uniformly seeding the endothelial cells in the lumen of the scaffold and enhancing the seeding effectiveness. However, the results achieved have not been entirely satisfactory.

WO 03/082145 describes materials and molding technique for making complex, living, vascularized tissues for organ and tissue replacement with polymer scaffolds and semipermeable membranes.

WO 2018/063099 describes a device for patterning extracellular matrix (ECM) hydrogel with a first layer surface characterized by microchannel, and a second layer comprising a loading channel in fluid communication with loading ports to receive an ECM hydrogel wherein the ECM hydrogel is confined to form a perfusable channel and therefore to provide a improved and efficient method for patterning microchannel of different vascular geometries in ECM hydrogel.

Static seeding and dynamic seeding currently represent the main endothelial cell seeding methods. The simplest static method consists in pipetting a cell suspension directly on the luminal surface of the scaffold followed by a short incubation step on a Petri dish. This method strongly depends on the operator and it is complicated by the difficulty to obtain a uniform endothelial layer.

Vastly based on rotation, vacuum, electrostatic or magnetic forces, the dynamic methods on the contrary increase cell seeding effectiveness, uniformity and adhesion. Some of these dynamic principles could be directly transferred and paired with perfusion bioreactors, allowing a reduction in the handling of the scaffold. In this case, the scaffolds can be immediately seeded once housed in the bioreactor chamber.

Other research groups instead use the dynamic seeding method through a continuous injection of a cell suspension using a syringe pump, or through a continuous perfusion of the lumen of the scaffold seeded with a growth medium using a peristaltic pump, after injecting the endothelial cell suspension. These methods require higher volumes of cell suspension with respect to a pipetting/rotation seeding method due to the need of also filling the volume of the perfusion piping as well as the volume of the injection syringe, revealing possible limitations with reference to reducing costs (growth cells and media).

A completely different technique with respect to the ones described previously is based on dripping the cell suspension in the lumen of a scaffold. In this case, the main drawback lies in a low initial adhesion of the cells and a low reproducibility of the method given that it strongly depends on the operator.

Furthermore, the choice of a method for connecting a perfusion circuit (perfusion method), required for the perfusion of a scaffold, mainly tubular, to the bioreactor-scaffold system must be adapted to the experimental setup.

Considering the landscape regarding the methods for seeding and connecting a perfusion circuit (perfusion method) mentioned above, considerable limits and drawbacks still exist.

Therefore, there clearly arises the need to provide a process comprising a method for seeding and connecting the perfusion circuit (perfusion method) to the bioreactor-scaffold system that is reproducible, reliable and effective, especially considering the applicability of said process for GLP (Good Laboratory Practice)-approved Tissue engineering laboratories whose objective focuses on advanced preclinical tests and clinical tests.

There arises the need to develop a process for the production of engineered vascular tissues/constructs having a continuous (i.e. having a monolayer of confluent cells) and functional endothelium, wherein said process is simple, quick, effective, independent from the operator, well defined, highly reproducible and reliable, comprising: (1) a method for seeding cells, mainly endothelial cells, in the lumen of a scaffold, that guarantees the homogeneity and the uniform adhesion of the endothelial cells, with the elimination of air bubbles in the scaffold; (2) a method for connecting the perfusion circuit to the bioreactor-scaffold system (perfusion method) capable of guaranteeing sterility and maintaining the absence of air bubbles in the assembled system consisting of the perfusion circuit and bioreactor-scaffold system.

There also arises the need of being able to develop in vitro engineered vascular tissues/constructs comprising a continuous and functional endothelium for conducting advanced preclinical and clinical tests, so as to avoid using laboratory cavies to conduct preclinical and clinical tests. Therefore, there arises the need for a procedure that is reliable, effective and reproducible for the industrialisation of said in vitro engineered vascular tissues/constructs.

As observed above, the process for developing and approving medicinal products, such as for example medical devices and drugs, normally requires conducting numerous preclinical tests to be conducted in- vitro, ex-vivo and in-vivo on animals prior to conducting clinical testing on humans. Despite animal testing offering an in vivo model for the test of medicinal products, the anatomical structure, the physiology and the blood composition considerably differ from those of humans and the results achieved on the animal model may considerably differ from those achieved in human testing.

Furthermore, besides being technically complex, causing diseases (such as for example vascular dysfunctions, aneurysms or stenosis) using animal models is widely ethically inadmissible. Thus, the in vivo test on animals cannot be conducted to verify whether the medicinal product is effective at treating given diseases. To confirm the scientific willingness and policy towards minimising in vivo testing on animals, since 1987 the 3R (Replace, Reduce, Refine) Research Foundation has been promoting the development of methods alternative to the animal model and has been creating awareness among the public and scientific community around animal testing only in the absence of alternative methods so as to meet the "technical" requirement, with the aim of reducing the number of laboratory animals and suffering inflicted on the animals to the minimum. Methods alternative to animal testing represent significant progress not only from an ethical point of view as regards treating animals more responsibly but above all as concerns the possibility of developing models that are reliable, reproducible and that allow to simulate the "human" environment to the uttermost. Computerised simulations and in vitro cell culture for the preliminary analysis of some characteristics of medicinal products have been created in recent years to this end.

In compliance with the 3R principle and with the aim of overcoming the anatomical/physiological limitations of animal models, it would be useful to have dysfunctional engineered vascular models capable of reproducing the diseases (such as for example stenosis, aneurysms) and "special" vascular structures as testing platform for medical devices and drugs prior to the use thereof in humans. The use of an in vitro system capable of replicating, in a standardised manner, human anatomical structures and the in-vivo environment would not only allow to accelerate the research and development of new medicinal products, but also to obtain more reliable responses on the safety and on the effectiveness of the tested products with respect to the animal model.

The Applicant met the aforementioned needs.

Forming an object of the present invention is an in vitro model of a substantially tubular-shaped vascular structure having dysfunctional anatomical and physiological characteristics, simulating the vascular structure of a healthy subject whose vascular structure has been damaged or deformed or deteriorated due to a damage selected from among the group comprising or, alternatively, consisting of aneurysm, stenosis, sclerosis plaques, forms of tumours or cardiomyopathies having the characteristics as claimed in the attached claims.

Furthermore, forming an object of the present invention is an in vitro model of a substantially tubular shaped vascular structure having dysfunctional anatomical and physiological characteristics simulating the same vascular structure of a healthy subject whose vascular structure has been damaged or deformed or deteriorated due to a damage selected from among the group comprising or, alternatively, consisting of aneurysm, stenosis, sclerosis plaques, forms of tumours or cardiomyopathies, wherein said model comprises or, alternatively, consists of one or more biocompatible porous polymeric supports (" scaffolds ") capable of promoting a cell adhesion and growth, wherein said scaffold is seeded with endothelial cells that cover a lumen of the scaffold and constitute an endothelium having a monolayer of confluent cells, said scaffold being provided with deformities or defects on a tubular structure thereof, having the characteristics as claimed in the attached claims.

Preferably said in vitro model has a vascular structure that was selected from among the blood vessels or valves of the central or peripheral circulatory system; preferably arteries, veins, capillaries, aortic or mitral valve. Preferably, said in vitro model is a dysfunctional vascular model. Preferably, said vascular structure is synthetic vascular structure. Preferably, said scaffold consists of electrospun silk fibroin, copolymers of polyglycolic acid/polylactic acid (PGA/PLA) or copolymers of polyglycolic acid/polycaprolactone (PGA/PCL). Preferably, said in vitro model comprises or, alternatively, consists of one or more scaffolds seeded with endothelial cells, and optionally muscle cells. Preferably, said deformities or said defects of the tubular structure comprise bifurcations, curvatures, elbows, constrictions, dilatations or combinations thereof. Forming an object of the present invention is a method for testing a medical device or a drug with the aim of verifying the effectiveness and safety thereof prior to the in vivo use thereof on humans or animals, said method comprising the following steps:
- preparing a substantially tubular-shaped scaffold having the dysfunctional anatomical and physiological characteristics suitable to simulate a damage or a deformation or a deterioration due to an aneurysm, stenosis, sclerosis plaques, forms of tumours or said scaffold having deformities or defects on the tubular structure thereof which are curvatures, elbows, constrictions, dilatations; said scaffold consisting of electrospun silk fibroin, copolymers of polyglycolic acid/polylactic acid (PGA/PLA) or copolymers of polyglycolic acid/polycaprolactone (PGA/PCL);
- seeding at least part of the internal lumen of said scaffold with endothelial cell lines so as to obtain a continuous and homogeneous layer of seeded endothelial cells (seeding method), optionally seeding at least part of the outer surface of said scaffold with muscle cell lines;
- promoting the growth of said endothelial cells, and optionally said muscle cells, up to obtaining a single continuous anc uniform layer of endothelial cells, to obtain said in vitro model;
- introducing into said in vitro model a medical device or a drug subject of test, and
- allowing the circulation (perfusion model) in said in-vitro model comprising said medical device or drug of a human whole blood sample, artificial blood or derivatives thereof so as to evaluate the behaviour and the interaction of said medical device or drug with said human whole blood sample, artificial blood or derivatives thereof.

Forming an object of the present invention is a model for in-vitro simulation of the behaviour of dysfunctional human vessels comprising or, alternatively, consisting of vessels affected by aneurysm, stenosis or sclerosis plaques, as an instrument for testing medical devices and drugs with the aim of verifying the effectiveness and safety thereof prior to use thereof on humans, having the characteristics as claimed in the attached claims.

Also forming an object of the present invention is providing in vitro vascular structure models, mainly substantially tubular-shaped, having, depending on the need, dysfunctional anatomical and physiological characteristics with respect to the healthy human vascular structure, such as, by way of non-limiting example, aneurysms, stenosis, sclerosis plaques, having the characteristics as claimed in the attached claims.

Forming another object of the present invention are dysfunctional vascular models comprising or, alternatively, consisting of one or more scaffolds seeded with suitable selected cells, having the characteristics as claimed in the attached claims.

Forming another object of the present disclosure is a method for providing said dysfunctional vascular models using scaffolds seeded with suitable selected cells, having the characteristics as claimed in the attached claims. Such dysfunctional vascular structures include: (i) a scaffold consisting of biocompatible material suitable to be electrospun, such as for example silk fibroin, or molten in moulds or printed using bioprinters such as for example polylactic acid, polycaprolactone, etc. The structure of the scaffold is suitable to allow the seeding of endothelial vascular cells in the scaffold and to allow the through-flow of fluids such as growth medium for sustaining the cells, blood (artificial or whole human).

A different embodiment of the structure of the scaffold described in the previous point provides for the possibility of seeding, on the outer surface of the scaffold, as a function of the characteristics to be tested in the medicinal product subject of the test, muscle cells or nervous cells, providing for a weft that promotes the orientation of said cells so as to be able to simulate the vasoconstriction or vasodilation depending on the mechanical, pharmacological or chemical stimulus to which the model is subjected.

In the scaffold, for example in order to simulate clots, sclerosis plaques, thrombosis or stenosis, blood components (such as for example platelets, cholesterol, erythrocytes, etc.).

Still with the aim of simulating a dysfunction of the vascular structure, the scaffolds can be made having deformities or defects on the tubular structure thereof, such as for example, bifurcations, curvatures, elbows, constrictions, dilatations.

The hydraulic circuit and the bioreactor into which the scaffold is inserted is outlined hereinafter in the present description to which reference shall be made with the help of the attached drawings. The circuit, already as provided, allows the perfusion of the seeded cells on the scaffold with growth medium so as to allow the survival and growth thereof, with the possibility of passing to a pulsatile regime upon replacing the growth medium with blood (whole or diluted with growth medium or with eluate of the product to be tested), or with artificial blood, or with growth medium diluted with eluate of the product to be tested. The circuit allows - by means of suitable valve upstream of the bioreactor - to introduce the medicinal product (drug, medical device) into the scaffold.

The dysfunctional scaffolds subject of the present invention and obtained by means of the method described herein were tested using:
Blood analysis (blood alterations caused by the dysfunction of the vessel and/or by the product used for treating the dysfunction of the vessel) or hemocompatibility of the medicinal product.

Biocompatibility of the medicinal product used for treating vascular dysfunction such as for example stents, devices for the embolization of the aneurysms, catheters, heart valves, drugs, etc.

Verifying functionality, feasibility, or appropriateness of the design of the medicinal product at correcting the vascular dysfunction. Furthermore, after a long and intense research and development activity, the Applicant created a process comprising a seeding method and a method for connecting the perfusion circuit to the bioreactor-scaffold system, having the characteristics as claimed in the attached claims.

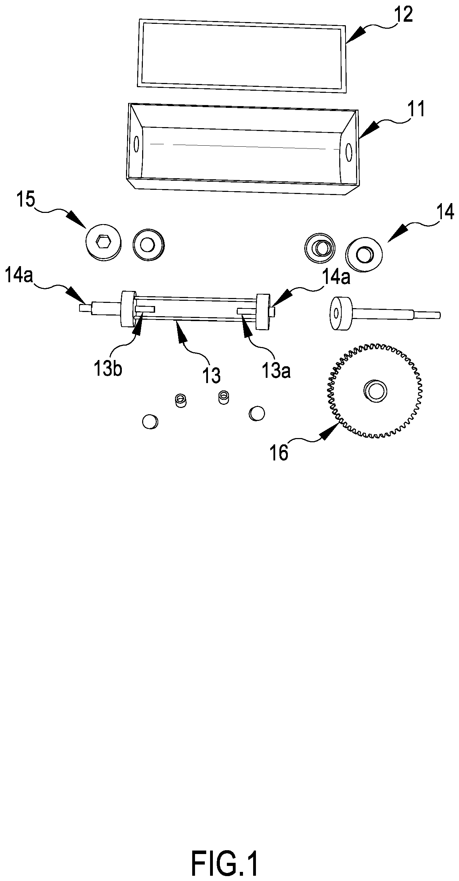

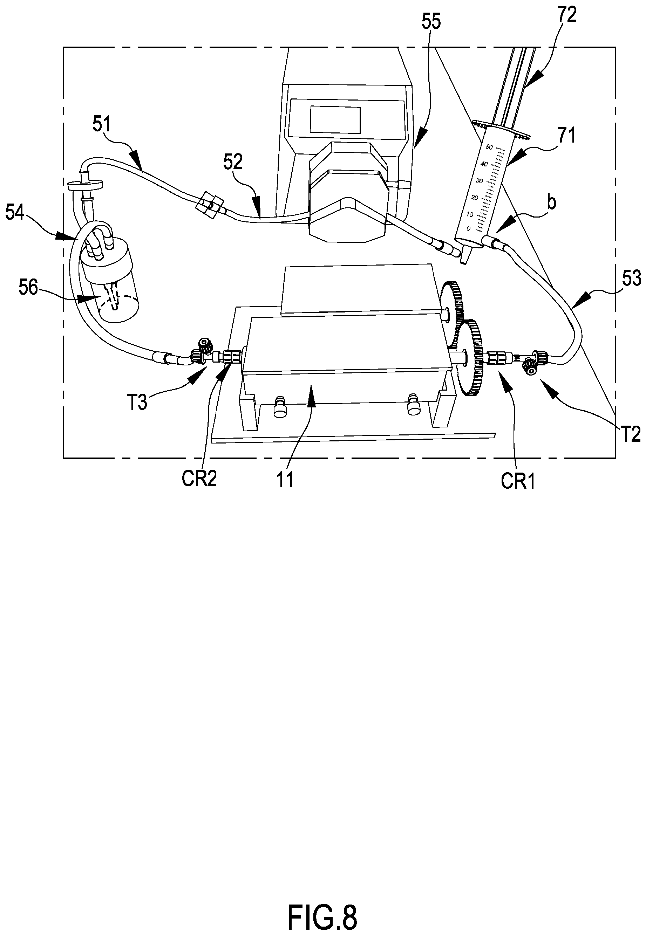

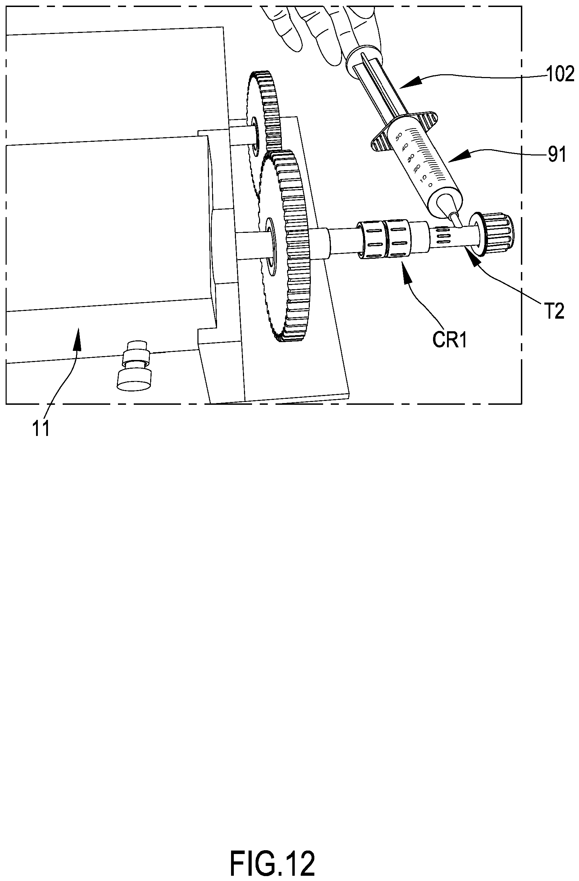

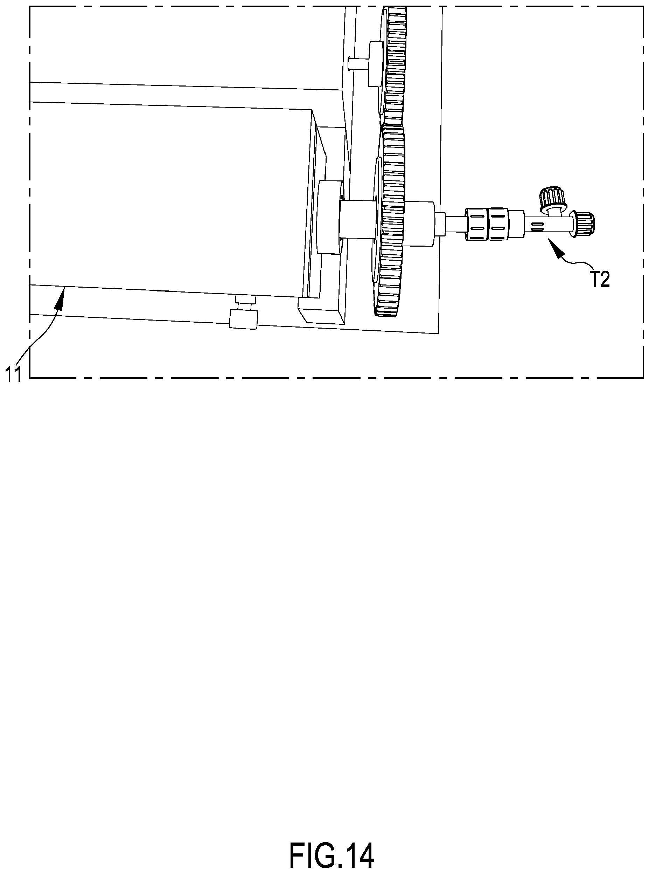

Preferred embodiments of the present invention will be clear from the detailed description that follows. Figures 1-27 are outlined hereinafter in the present description.

In the context of the present invention the expression continuous and functional endothelium is used to indicate an endothelium, with physiological-like behaviour, wherein the endothelial cells are adjacent to each other, adhered to the scaffold and expressing markers typical of the endothelial cells, such as for example Von Willebrand factor (VWF), cluster of differentiation 31 (CD31), vascular cell adhesion molecule 1 (VCAM-1). In particular, the expression continuous endothelium is used to indicate an endothelium having a monolayer of confluent cells.

In the context of the present invention the expression scaffold is used to indicate a biocompatible porous polymeric medium capable of promoting the cell adhesion and growth, endothelial cells in this case. The polymeric scaffold can be of synthetic or natural origin and consist of only one polymer or copolymers (entirety of polymers), chosen from electrospun silk fibroin or copolymers of PGA/PLA (polyglycolic acid/polylactic acid) or PGA/PCL (polyglycolic acid/polycaprolactone).

In the context of the present invention the expression "confluence" refers to a surface of the scaffold (in particular an inner surface or lumen of such scaffold) which is covered by adherent cells. In particular, so- called "confluent cells" have a confluence equivalent to or greater than 90%, preferably comprised between 90% and 100%, even more preferably comprised between 95% and 100%, hence substantially the entire surface of the scaffold is covered by adherent cells and there is no more surface left available on the scaffold so that the cells can grow as a monolayer.

In the context of the present invention the cells constituting an endothelium of a vascular tissue are defined as endothelial cells. Examples of endothelial cells are the FIAOECs (human aortic endothelial cells), FICAECs (human coronary artery endothelial cells), FIMEVECs (human dermal microvascular endothelial cells), or FIUVECs (human umbilical vein endothelial cells).

In the context of the present invention, a scaffold having the lumen mainly covered by functional endothelial cells following the in vitro endothelisation process is defined as engineered vascular construct. Said endothelial cells covering the lumen of the scaffold constitute a continuous endothelium, i.e. an endothelium having a monolayer of confluent cells.

In the context of the present invention, the cell growth and maintenance fluid, specific for each type of cell, is defined as growth medium. In particular, as concerns endothelial cells used in this specific case (HUVECs - Human Umbilical Vein Endothelial Cells, purchased from Sigma Aldrich, code 200-05n), the growth medium is Endothelial Growth Medium (EGM, Sigma Aldrich, 211-500). EGM contains fetal bovine serum (2%), adenine (0.2 pg/ml), ammonium metavanadate (0.0006 pg/ml), amphotericin B (0.3 pg/ml), calcium chloride 2H20(300 pg/ml), choline chloride (20 pg/ml), copper sulphate 5H2O (0.002 pg/ml), trioptic acid DL-6,8(0.003 pg/ml), folinic acid (calcium) (0.6 pg/ml), heparin (4 pg/ml), hydrocortisone (2 pg/ml), L-aspartic acid (15 pg/ml), L-cysteine (30 pg/ml), L-tyrosine (20 pg/ml), manganese sulphate monohydrate (0.0002 pg/ml), ammonium molybdate 4H2O (0.004 pg/ml), nicotinamide (8 pg/ml), nickel chloride 6H2O (0.0001 pg/ml), penicillin (60 pg/ml), phenol red sodium salt (15 pg/ml), potassium chloride (300 Mg/ml), putrescine dihydrochloride (0.0002 pg/ml), pyridoxine hydrochloride (3 pg/ml), sodium metasilicate 9H2O (3 pg/ml), sodium sulphate 7H2O (200 pg/ml), sodium selenite (0.01 pg/ml), streptomycin sulphate 100 pg/ml), thiamine hydrochloride (4 pg/ml), and zinc sulphate 7H2O (0.0003 pg/ml). The fresh growth medium is the sterile medium not used previously, directly supplied by the manufacturer. The expression hot growth medium is used to indicate that the growth medium was previously heated at a temperature comprised in the range between 30°C and 45°C, preferably at 37°C.

The process subject of the present invention comprises a seeding method and a method for connection between a bioreactor and a scaffold perfusion circuit (perfusion method), preferably tubular scaffolds, for engineering a vascular tissue with ensuing production of vascular grafts engineered (vascular constructs/tissues) for testing medicinal products. Said process, comprising the seeding method and the method for connecting a perfusion circuit for a bioreactor-scaffold system (perfusion method), advantageously guarantees the accurate removal of air bubbles from the system described hereinafter and, thus, it guarantees maximum reproducibility of the process. Furthermore, reducing the risk of the air bubbles coming into contact with the endothelial cells allows to prevent damaging the endothelial cells and it allows to obtain a confluent monolayer of endothelial cells adhered onto the lumen of the scaffold (continuous and functional endothelium). In this specific case, such seeding method and method for connecting a scaffold perfusion circuit (perfusion method) is applied onto a scaffold preferably tubular electrospun silk fibroin in a bioreactor for the perfusion.

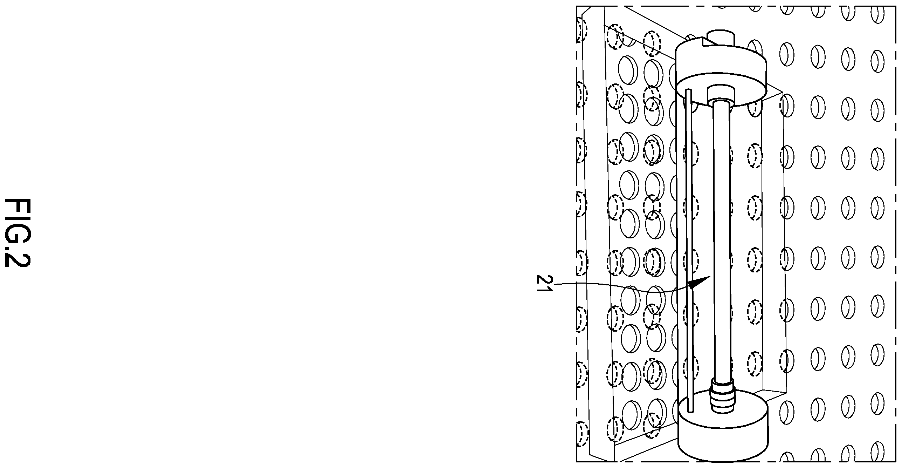

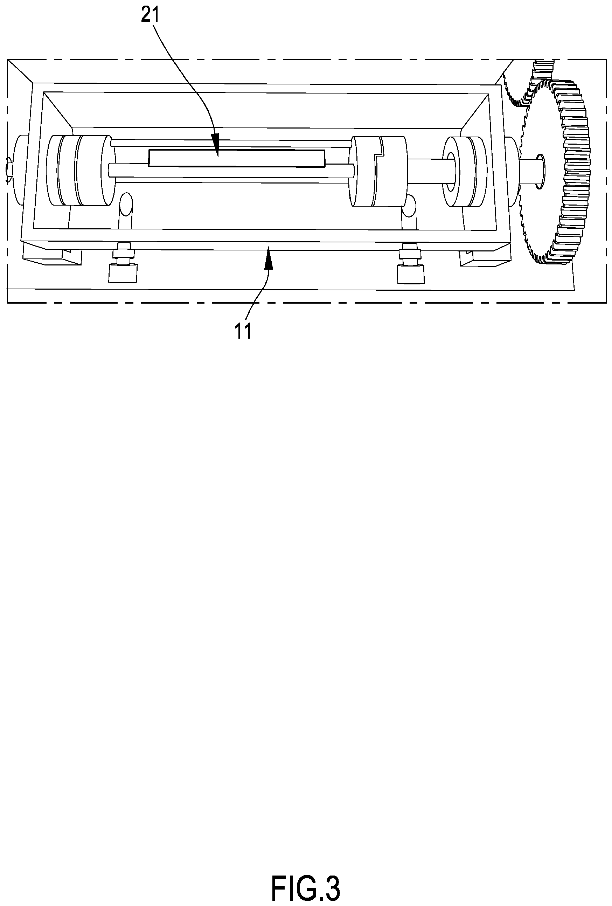

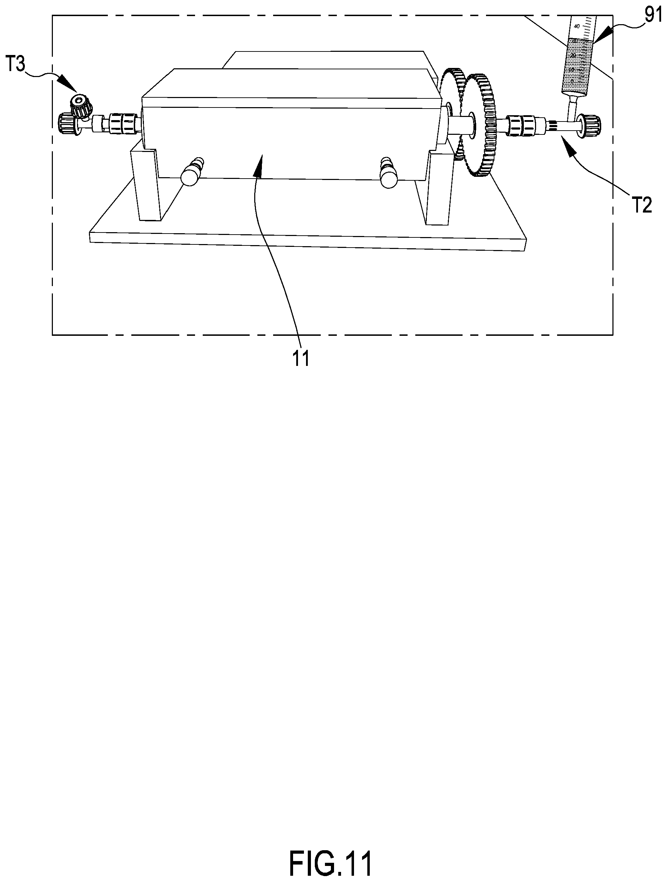

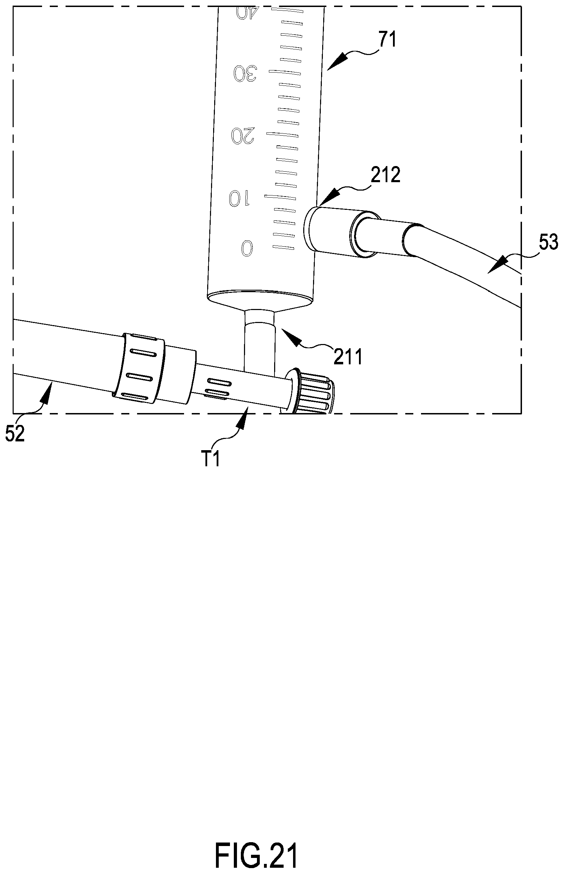

The process of the present invention allows to overcome the limitations of the models currently available and meeting the 3R requirements in that it offers a valid alternative to using animal models. Forming an object of the present invention is a process for producing an engineered vascular tissue or construct, preferably a scaffold (Fig. 2, 21) having a lumen covered with a functional and continuous endothelium having a confluent cell monolayer, preferably usable for testing medicinal or veterinarian products wherein said process comprises applying:
- a method for seeding an endothelial cell culture in the lumen of a scaffold (21) to obtain a seeded scaffold (21); said seeded scaffold (21) being present in a bioreactor (11), to obtain a bioreactor (Fig. 3, 11)-seeded scaffold (21) system;

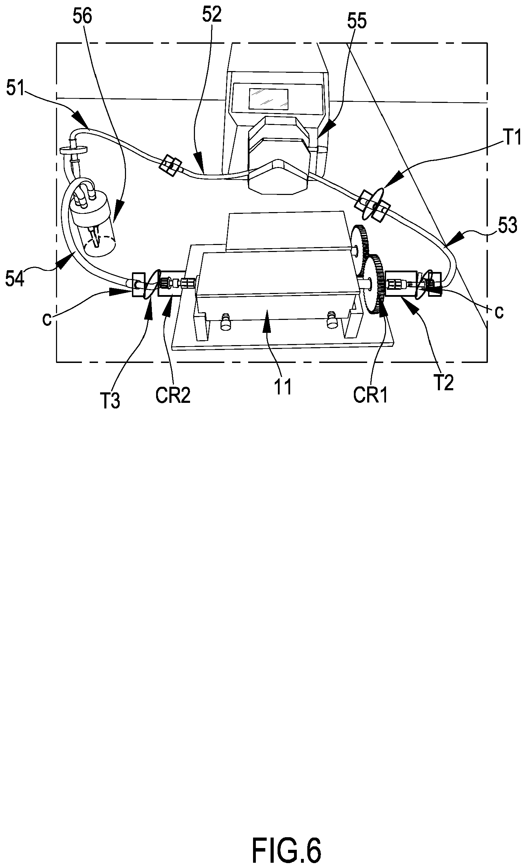

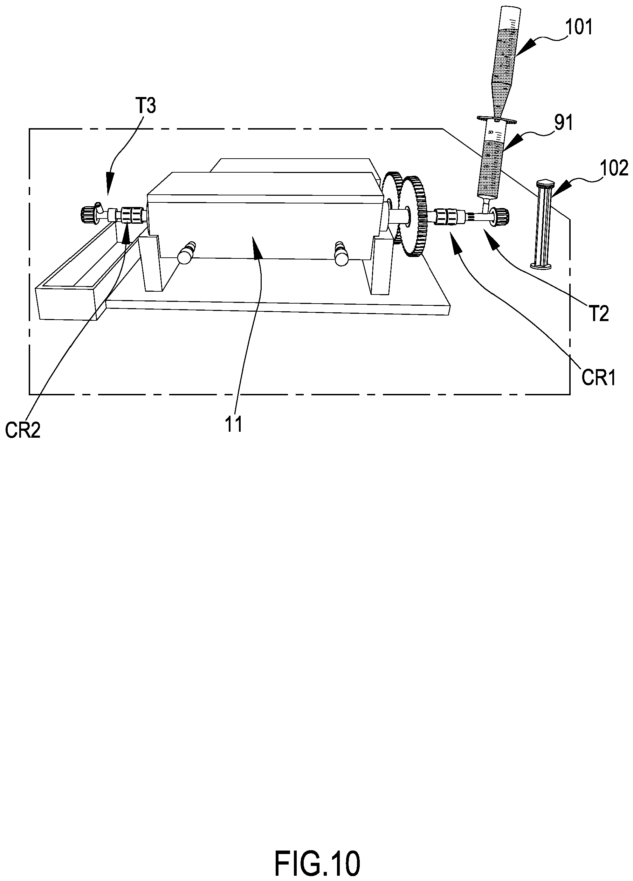

wherein said seeding method comprises the steps of:
   - releasing said endothelial cell culture in form of a cell suspension comprising a fresh growth medium and endothelial cells in a container (Fig. 10, 91) mounted on a T-shaped connector (Fig. 10, T2) arranged upstream of the bioreactor (11) by means of a rotary connector (Fig. 10, CR1); followed by
   - releasing said endothelial cell culture in the lumen of the scaffold (21) present in the bioreactor chamber (11) with a continuous flow such that the flow speed allows said cell suspension to drip into the T-shaped connector (T2) without generating air bubbles and pushing the air bubbles present in the lumen of the scaffold (21) towards an opening of a T-shaped connector (Fig. 10, T3) arranged downstream of the bioreactor (11) allowing the outflow thereof;
and, subsequently,
   - a method for perfusion - with a fresh growth medium having a temperature comprised in the range between 30°C and 45°C, preferably at 37°C - of the endothelial cells present in the lumen of said seeded scaffold (21); said perfusion method being obtained by connecting a perfusion circuit (Fig. 6; 51 , 52, 53, 54, 55, and 51-56 or 51-57 and BT) to said bioreactor (11)-seeded scaffold (21) system;
wherein said perfusion method comprises a step of
   - partly filling an element for removing the air bubbles (71 or BT) present in the perfusion circuit with said fresh growth medium, wherein said element for removing the air bubbles (71 or BT) comprises a chamber, a cap that closes said chamber, an access with inflow function (211) and an access with outflow function (212), wherein said chamber of the element for removing the air bubbles (71 or BT) has a volume and wherein a first part of said volume is filled with said fresh growth medium and wherein a second part of said volume is filled with air, said second part of said volume having the function of trapping the air bubbles present in said fresh growth medium which flows through said access with inflow function (211) and said access with outflow function (212).

With the aim of illustrating preferred embodiments, the proposed technical solution represented by the process subject of the present invention is, for ease of comprehension, divided into the two methods which are described in detail separately hereinafter: (1) method for seeding a cell culture in the lumen of a scaffold, preferably a tubular scaffold; (2) method for connecting a perfusion circuit to a bioreactor-scaffold system (perfusion method).

### (1) Method for seeding a cell culture in the lumen of a scaffold according to a preferred embodiment.

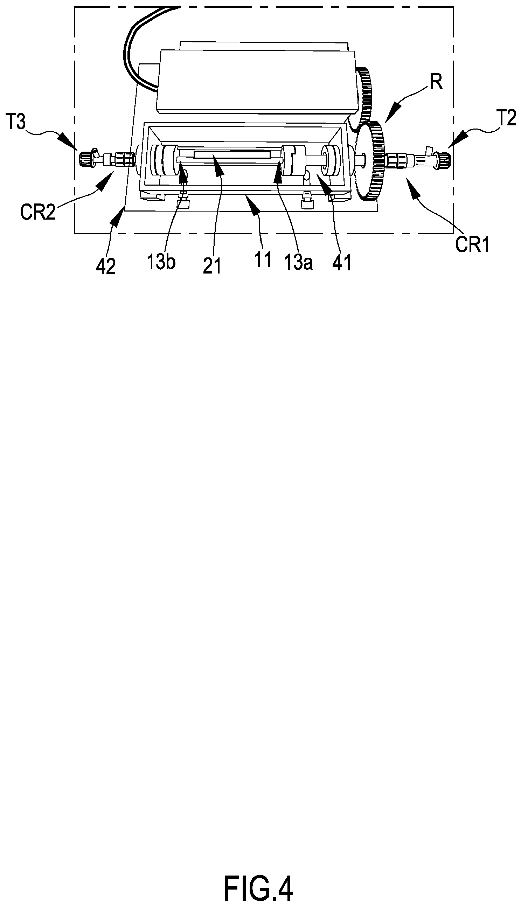

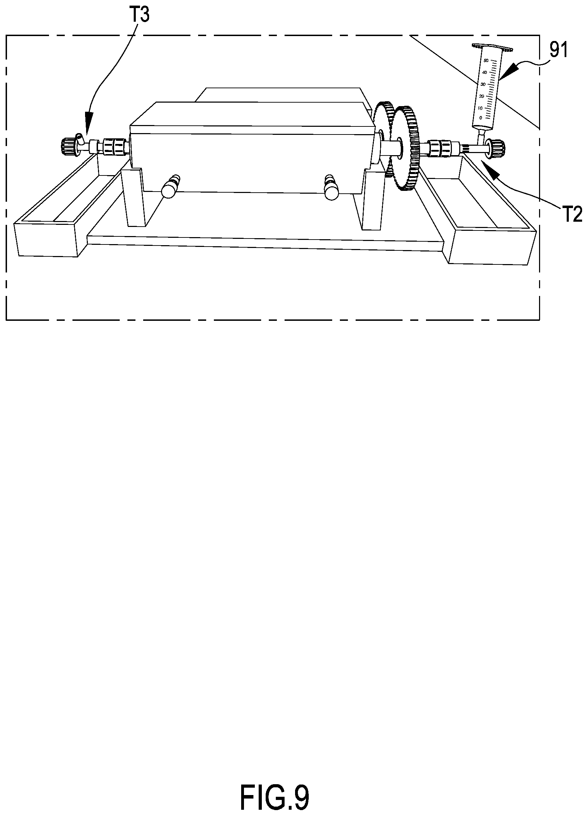

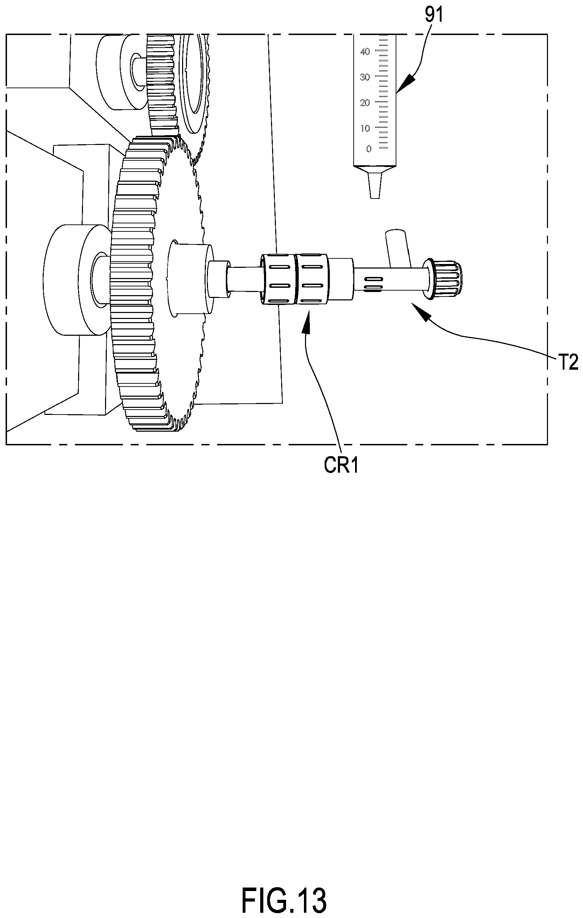

The method for uniform seeding of endothelial cells for a bioreactor-scaffold system, comprises a plurality of steps carried out sequentially and under sterile conditions:
1.1 A scaffold (Fig. 2; 21), preferably a tubular scaffold, for example an electrospun silk fibroin tubular scaffold, mounted on the grips of the scaffold-holder (Fig. 1 ; 13, 13a, 13b) is housed in the bioreactor chamber, to obtain a bioreactor-scaffold system. Applied at both ends of the bioreactor (upstream and downstream) are rotary connectors (Fig. 4; CR1, CR2) and T-shaped connectors (Fig. 4; T2, T3).
   In the context of the present invention the expression bioreactor-scaffold system is used to indicate the assembly of the bioreactor and the scaffold (Fig. 3; 11, 21), preferably a tubular scaffold such as for example an electrospun silk fibroin tubular scaffold, which is housed and fixed by means of grips of the scaffold-holder(Fig. 1; 13, 13a, 13b) which is arranged in the bioreactor. The scaffold, preferably tubular, is fastened to the grips of the scaffold-holder (which is internally hollow so as to allow the perfusion of the scaffold) with self-fastening strips, after having protected the scaffold, an electrospun silk fibroin in this case, with a sterilised teflon tape.
   The insertion of the scaffold-holder 13 into the bioreactor 11 occurs in a manner such that the inlet upstream of the bioreactor coincides (Fig. 4; CR1 , 41) with an end of the scaffold (Fig. 4; 13a) and the opening downstream of the bioreactor chamber (Fig. 4; CR2, 42) coincides with the other end of the scaffold (Fig. 4; 13b). In this manner, the scaffold 21 is perfectly coaxial with respect to the perfusion path generated by the internally hollow scaffold-holder. The larger axis according to which the scaffold mounted in the bioreactor is oriented is defined as the longitudinal axis.
1.2 The scaffold is preconditioned using fresh growth medium injected into the lumen of the scaffold which is housed in the bioreactor, using a syringe with a luer-lock connector which is engaged to one of the two ends of the bioreactor by means of a T-shaped connector (Fig. 9; T2). Subsequently, the open ends of the connectors arranged upstream and downstream of the bioreactor are closed using caps so as to avoid the emptying of the lumen of the scaffold. Furthermore, the fresh growth medium is inserted into the bioreactor chamber until the scaffold housed therein is fully covered. In this manner, the scaffold housed in the bioreactor chamber is preconditioned, preferably for 1 hour to 25°C, using a fresh growth medium both internally (in the lumen) and externally.
1.3 After preconditioning, the scaffold inside the bioreactor preconditioned with the growth medium injected into the lumen is emptied preferably using a sterile pipette and the growth medium previously introduced into the chamber is eliminated preferably using a sterile pipette. The growth medium residues present in the connectors (rotated and T-shaped) arranged downstream and upstream of the bioreactor are eliminated with a vacuum using a pipette, without making the scaffold collapse.
1.4 The T-shaped connectors arranged upstream (Fig. 9, T2) and downstream (Fig. 9; T3) of the end of the bioreactor, with the scaffold therein mounted on the grips, are directed with the upper opening upwards (at 90° with respect to the plane in which the bioreactor-scaffold system lies). Subsequently, the lateral opening of the T-shaped connectors arranged downstream (T3) and upstream (T2) of the bioreactor is capped. Mounted on the opening facing upwards of the T-shaped connector (Fig. 9; T2) arranged upstream of the bioreactor is a container, preferably a syringe (Fig. 9; 91) with a luer-lock connector with capacity for example of 5 ml without the plunger thereof. The opening of the T-shaped connector (Fig. 9; T3) arranged upstream of the bioreactor remains open instead (Figure 9).
1.5 Using a pipette, preferably a sterile plastic pipette with capacity of for example 25 ml (Fig. 10; 101), is drawn from a container prepared in which is a cell suspension consisting of fresh growth medium and endothelial cells (e.g. FIUVECs). Subsequently, the drawn cell suspension is released into the container or into the syringe (Fig 10; 91) mounted on the T-shaped connector element (Fig. 10; T2) upstream of the bioreactor through the element (Fig. 10; CR1). The cell suspension must be released, using the pipette (Fig. 10; 101), with capacity of for example 25 ml, with a continuous flow so that the flow speed allows the cell suspension to drip into the T-shaped connector (Fig. 10; T2) without generating air bubbles and pushing possible air bubbles present in the scaffold towards the opening of the T-shaped connector T3 arranged downstream (Fig. 10; T3) of the bioreactor 11 and, hence flow out (Figure 10).
1.6 When the cell suspension, loaded using a syringe, reaches the open end of the T-shaped connector T3 arranged downstream of the bioreactor without possible air bubbles, the T-shaped connector T2 arranged upstream of the bioreactor is closed using a cap (Figure 11).
1.7 Subsequently, the syringe (91) with the cell suspension residue is rotated by about 90° with respect to the plane on which it lies (Figure 12); in this position the plunger of the syringe (102) is re-inserted at the open end of the syringe (Figure 12) by inserting the insulating black part only so as not to create pressure inside the scaffold. Subsequently, the syringe (91) can be unscrewed from the T-shaped connector T2 upstream of the bioreactor without forming air bubbles (Figure 13) and the end of the connector is closed using a cap (Figure 14).
1.8 A hot fresh growth medium (as previously defined) is added into the bioreactor chamber until the seeded scaffold present in the bioreactor chamber is half-submerged in the growth medium.
1.9 A continuous rotation is then applied along the longitudinal axis of the scaffold, for example with a rotation speed comprised between 1.5 and 2 rpm, for 24 hours. The rotation allows the uniform cell adhesion on the lumen of the scaffold, allows the scaffold to remain continuously wet by the growth medium present in the bioreactor chamber and it allows the through-flow of the nutrients between the medium present in the chamber (outside the scaffold) and the cell suspension one seeded in the lumen of the scaffold.
1.10 Incubating, preferably for 24 hours at 37°C with 5% of CO2, the scaffold housed in the bioreactor chamber (under rotation).

Advantageously, the seeding method created by the Applicant allows to operate under sterility conditions. Furthermore, the present seeding method subject of the present invention is rapid, reproducible, and advantageously allows to prepare a scaffold having the lumen surface (internal) with endothelial cells homogeneously and uniformly adhered along the entire length of the scaffold (from the proximal part to the medial part up to the distal part). The present seeding method subject of the present invention allows to seed the cells eliminating both the air bubbles present in the bioreactor-scaffold system and the air bubbles that are formed, hence avoiding to damage the cells. Basically, each step of the present method is standardised and reproducible and it optimises the cost and the operating time.

### Experimental evidence of the seeding method

To prove the effectiveness of the seeding method subject of the present invention, the following analysis were conducted.

The viability of the cells adhered to the scaffold was assessed using an assay which uses resazurin (trade name Alamar Blue, name IUPAC 7-hydroxy-10-oxidophenoxazin-10-ium-3-one, CAS 550-82-3) as reagent. Such assay consists in a metabolic reaction that allows to quantify cell viability due to the oxidation-reduction of the indicator (resazurin) which is reduced to resofluorine, a pink fluorescent compound in the presence of reducing atmosphere of a vital cell. After 24 hours of adhesion, the seeded scaffold is removed from the grips. Subsequently, the scaffold is sectioned (cutting it) into three areas measuring about 2cm each depending on the distance from the site of injection of the cell suspension: proximal, medial and distal. Subsequently, each section is divided into 4 parts measuring about 1cm<2>. 3 samples each representing each region (proximal, medial, distal) of the scaffold with adhered endothelial cells were selected for the assay with resazurin. Each sample is positioned in a well of a 24-well dish and incubated with 1 ml of a 0.02 mg/ml resazurin sodium salt (Sigma Aldrich, R7017) solution with fresh growth medium preferably for 3 hours at 37°C with 5% of CO2. The reaction that is developed between the 0.02 mg/ml resazurin sodium salt solution with fresh growth medium and the scaffold sample (with the adhered endothelial cells) is analysed using the A.U. (arbitrary unit of fluorescence) detection at 590 nm by using a spectrofluorometer.

A further analysis conducted is the assessment of the amount of genomic DNA present in the cells adhered on the samples of the scaffold previously used for the assay with resazurin. The genomic DNA is extracted from the adhered cells by a scaffold through lysis and it is subsequently quantified using Quant- iT^{™} PicoGreen^{™} dsDNA Assay (P7589, Invitrogen, Molecular Probes) where the fluorescent stain of the nucleic acids (PicoGreen) allows - through a standard reference curve - to determine the concentration of genomic DNA in solution.

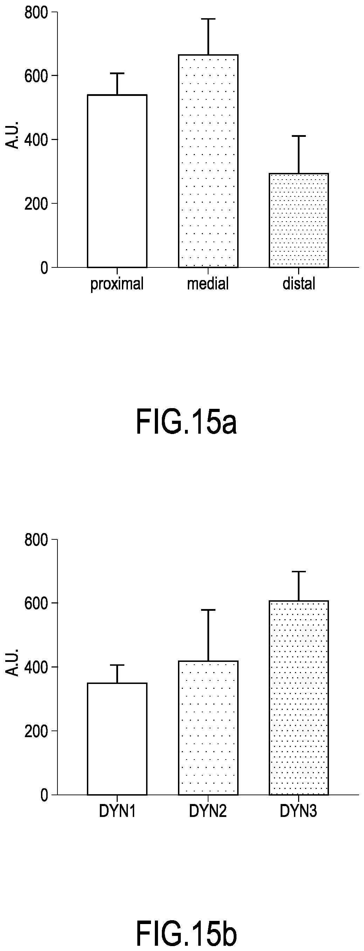

In Figures 15A and 15B represented in the chart are the values obtained using the assay with resazurin on samples representing each region (proximal, medial, distal) of a scaffold seeded with endothelial cells and incubated for 24 hours in three different experiments (named DYN1 , DYN2 and DYN3). The charts in Figures 15A and 15B show a good adhesion and viability of the endothelial cells.

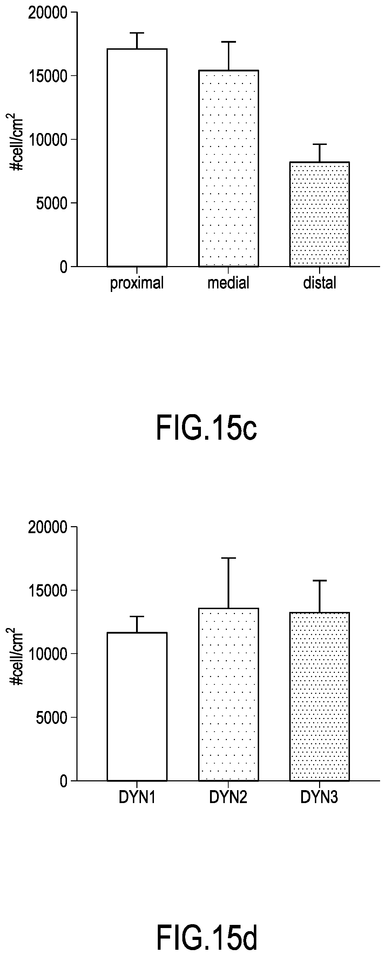

This data was confirmed by the quantification of the genomic DNA (Figures 15C and 15D) calculated considering that the genomic DNA content of an endothelial cell is of about 7 pg.

No significant cell viability difference was observed among the various proximal, medial and distal sections of the scaffolds seeded with endothelial cells. In particular, cell viability and the number thereof can be compared along the length (main axis of the scaffold) in the proximal, medial and distal portions thereof. With the aim of supporting this evidence, costaining was conducted using DAPI (4',6-Diamidino-2- Phenylindole, Dihydrochloride, D1306, ThermoFisher scientific) and Rhodamine-Phalloidin (R415, ThermoFisher scientific) on samples representing each region (proximal, medial, distal) of a scaffold seeded with endothelial cells and incubated for 24 hours. The two DAPI and Rhodamine-Phalloidin reagents are specific respectively for the nuclear detection and for actin filaments (F-actin), morphological components of a live cell, visible after the staining using a fluorescence microscope or a confocal microscopy. After 24 hours of culture, these results show that the endothelial cells are vital and distributed on the lumen of the scaffold in a uniform fashion. In particular, these results show a 90% cell confluence. Furthermore, conducted on samples representing each region (proximal, medial, distal) of a scaffold seeded with endothelial cells and incubated for 24 hours are gene expression analysis for markers typical of endothelial cells: for example, the Von Willebrand factor (VWF), cluster of differentiation 31 (CD31), vascular cell adhesion molecule 1 (VCAM-1). In order to conduct a gene expression evaluation, the total RNA is extracted from cells (endothelial in this case) and after reverse transcription at cDNA is quantified using a specific Taqman Gene Expression Assay (ThermoFisher Scientific) using the real-time PCR technique. Functional levels for gene expression of the markers listed previously are indicators of good functionality and viability of the cells adhered on the lumen of the scaffold.

Lastly, H&E"Haematoxylin and Eeosin" staining analysis is conducted on samples of a scaffold seeded with endothelial cells according to the present invention with the aim of evaluating the distribution of the cells and the morphology thereof, and an immunofluorescence assay for specific endothelial functionality markers. In conclusion, the present seeding method subject of the present invention revealed to be efficient in that it guarantees a homogeneous, uniform and reproducible seeding of vital endothelial cells along the entire lumen.

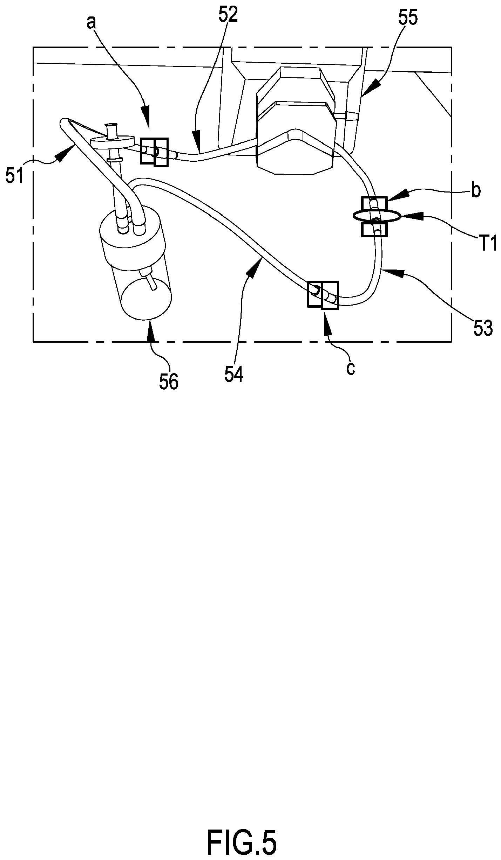

### (2) Method for connecting a perfusion circuit to a bioreactor-scaffold system (perfusion method) according to the first embodiment (Figures 5-8, 18-21).

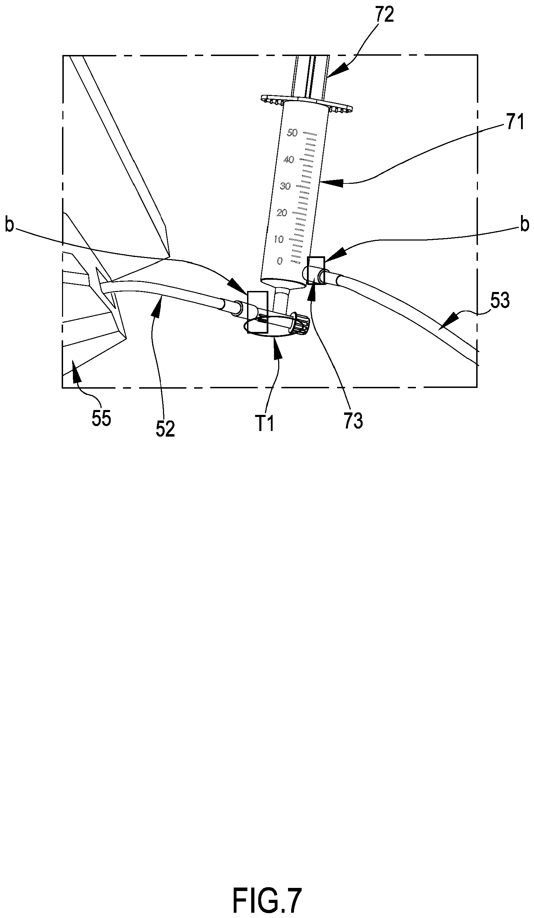

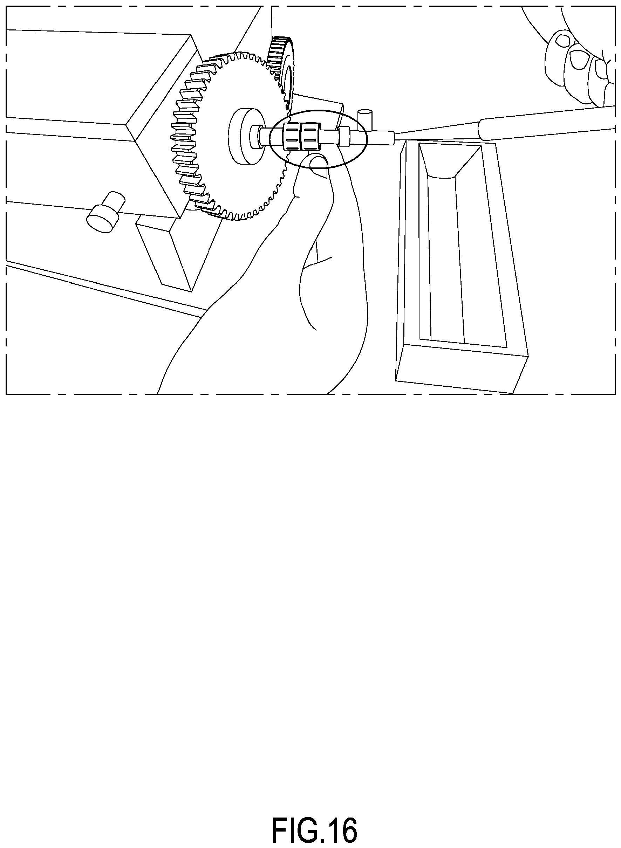

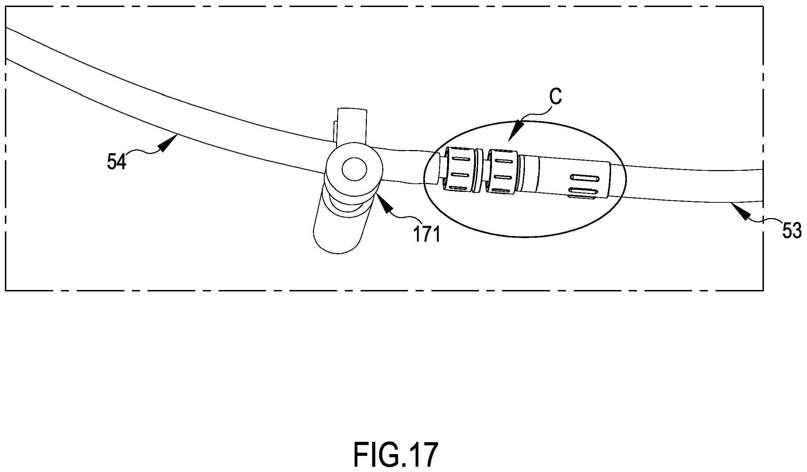

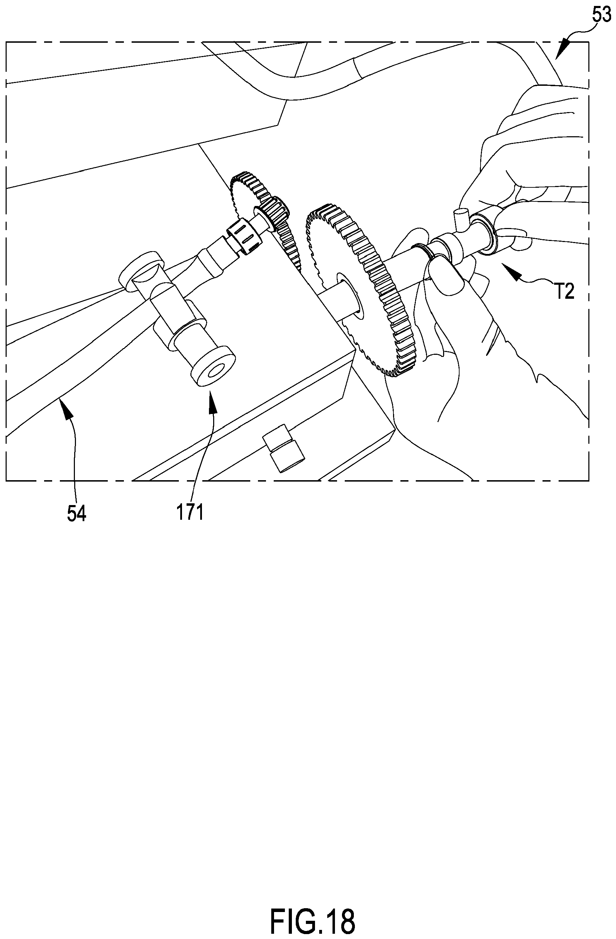

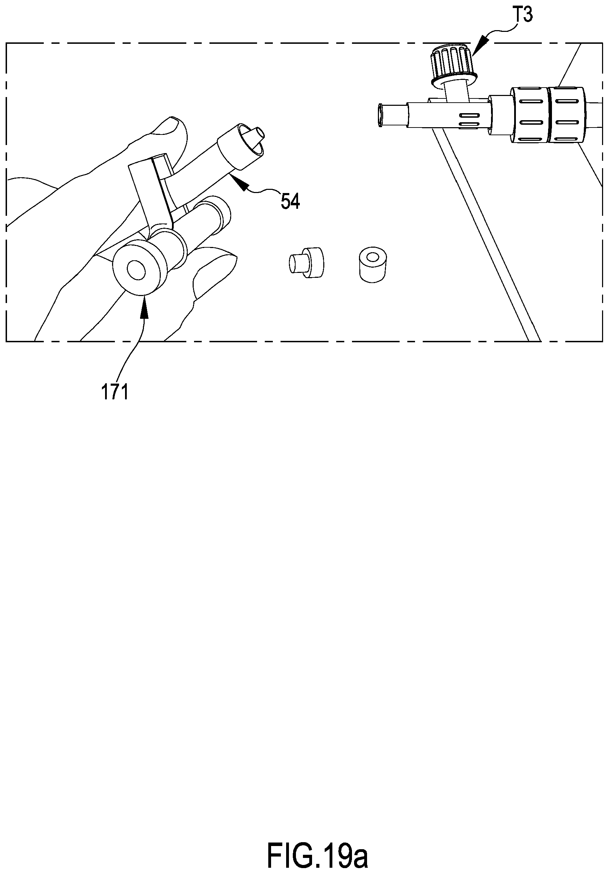

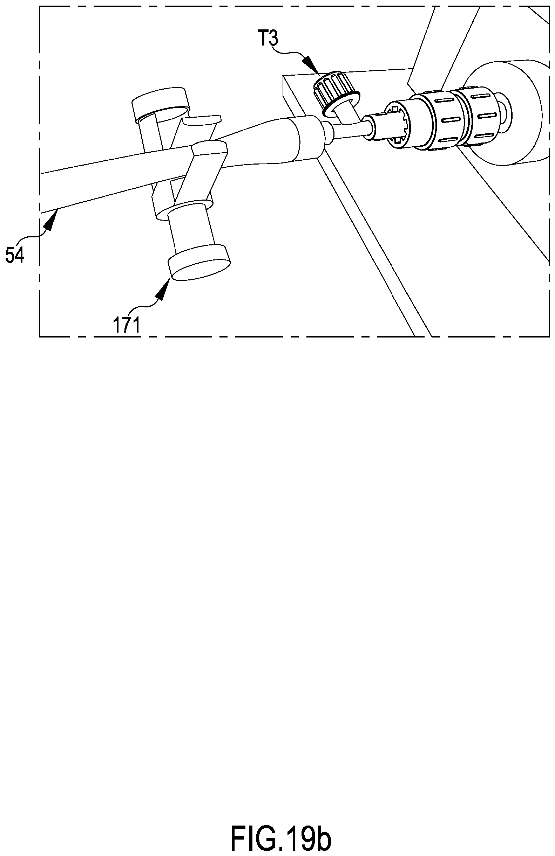

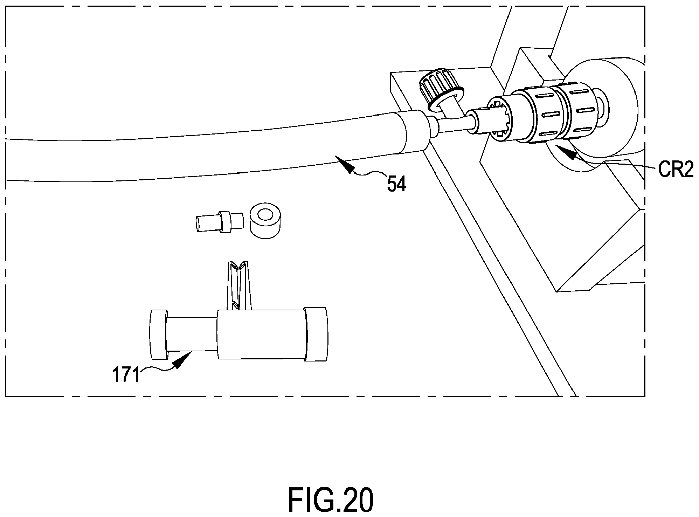

The connection method is based on the following sequential steps, subsequent to seeding (method for seeding a cell culture in the lumen of a scaffold according to the embodiment described above under point (1)) and at 24 hours from adhesion of the endothelial cells:
2.1 Place the tube or under-pump (Fig. 5; 52) of the closed perfusion circuit under the head of the peristaltic pump (Fig. 5; 55), which - upon activation - generates a peristaltic force capable of suctioning fluids, hot fresh growth medium in this case. The closed perfusion circuit is filled due to the suctioning, by the tube (Fig. 5; 51) of the perfusion circuit connected to the reservoir (Fig. 5; 56), of the hot fresh growth medium which is previously poured into the reservoir once closed. Fill the tubes of the perfusion circuit with the hot fresh growth medium until the fresh growth medium returns to the reservoir through the tube 54 (Fig. 5; 54) of the closed perfusion circuit. Place the bioreactor-scaffold system under the same sterility conditions as the perfusion circuit.
2.2 Open the upper and lateral ends of the T-shaped connector T2 arranged upstream of the bioreactor.
2.3 Upon removing the caps from the upper and lateral ends of the T-shaped connector T2 arranged upstream of the bioreactor, the possible creation of air bubbles is compensated by manually adding (preferably using a pasteur pipette) having the same volume as the hot fresh growth medium (Fig. 16).
2.4 Occlude the tube 54 of the closed perfusion circuit, preferably using a clamp (Fig. 17; 171) in a position proximal to the connector between the tube 54 and the tube 53 of the perfusion circuit (Fig. 17). Be careful to keep the head of the pump closed so as to prevent the emptying of the tube 53 of the closed perfusion circuit
2.5 Keep the tube 53 of the closed perfusion circuit in vertical position, unscrew the connector arranged between the tube 53 and the tube 54 of the perfusion circuit and preferably cap the tube 54 of the perfusion circuit using a cap.
2.6 Screw the tube 53 of the perfusion circuit to the open lateral end of the T-shaped connector T2 upstream of the bioreactor at a lateral access thereof. The connector upstream of the bioreactor must always be kept in vertical position (Fig. 18).
2.7 Open the T-shaped connector T3 downstream of the bioreactor by unscrewing the cap of the lateral opening. 2.8 Remove the cap from the connector of the tube 54 of the perfusion circuit (Fig. 19A) and screw it onto the lateral opening of the T-shaped connector downstream of the bioreactor (Fig. 19B).
2.9 Remove the clamp 171 which occludes the tube 54 of the perfusion circuit (Fig. 20).
2.10 Fill the element for the removal of the air bubbles (Fig. 21 , 71), defined with the technical expression of bubble trap in the context of the present invention. The bubble trap consists of an element represented by a chamber closed using a cap and having two accesses serving as an outflow and inflow. The bubble trap chamber contains a volume of liquid (hot fresh growth medium in this specific case) and a volume of air that traps possible air bubbles present in the perfusion liquid which flows through the two accesses of the bubble trap chamber.
   Fill the bubble trap with hot fresh growth medium so as to leave a given air volume and close the chamber as well as its accesses using the respective caps.
2.11 Connect the bubble trap to the perfusion circuit previously connected to the bioreactor-scaffold system as follows (Fig. 7):
   a. close the tube 53 of the perfusion circuit using a clamp arranged proximally to the connector which connects the tube 53 to the lateral end of the T-shaped connector T1 (arranged between the tube 53 and the tube 52 of the perfusion circuit) and unscrew it (Fig. 7; 52).
   b. preferably, close the tube 53 of the perfusion circuit using a cap. Such operation prevents the emptying of the tube 53 of the perfusion circuit.
   c. cap the lateral end of the T-shaped connector T 1 (arranged between the tube 53 and the tube 52 of the perfusion circuit) and open the upper end thereof.
   d. open the inflow access of the bubble trap chamber and connect it to the access of the upper end and arranged vertically with respect to the T-shaped connector T1.
   e. open the outflow access of the bubble trap and connect it to the tube 53 of the perfusion circuit, being careful not to twist the tube at all.
   f. keep the bubble trap in vertical position.
   g. remove the clamp 171 from the tube 53 of the perfusion circuit just connected to the bubble trap chamber. Start the pump and open the cap of the upper end of the T-shaped connector T2 upstream of the bioreactor so as to eliminate possible air bubbles formed in the tube 53 during the process preventing them from reaching the scaffold. The peristaltic force applied by the pump to the assembled system, consisting of the perfusion circuit and the bioreactor-scaffold system will allow the perfusion of the scaffold.
   h. after such verification, close the T-shaped connector T2 upstream of the bioreactor using the cap thereof.

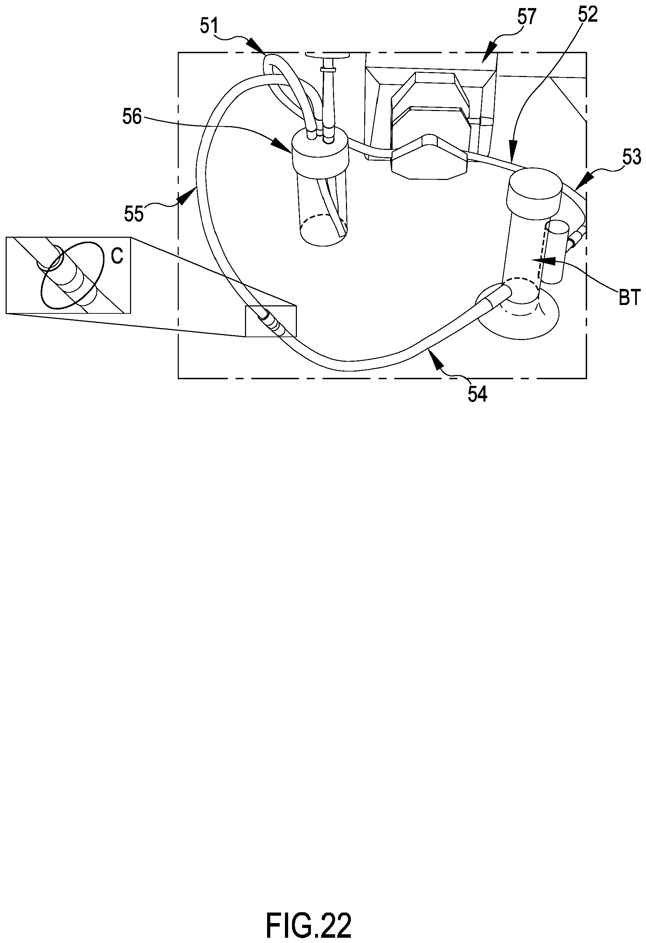

### (3) Method for connecting a perfusion circuit to a bioreactor-scaffold system (perfusion method) according to a second preferred embodiment (Figures 22-27).

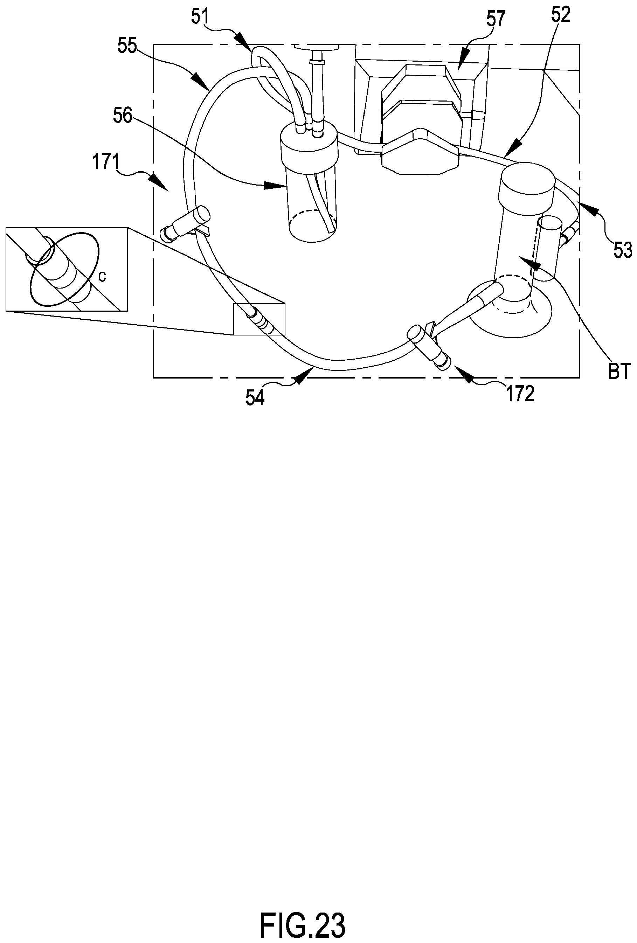

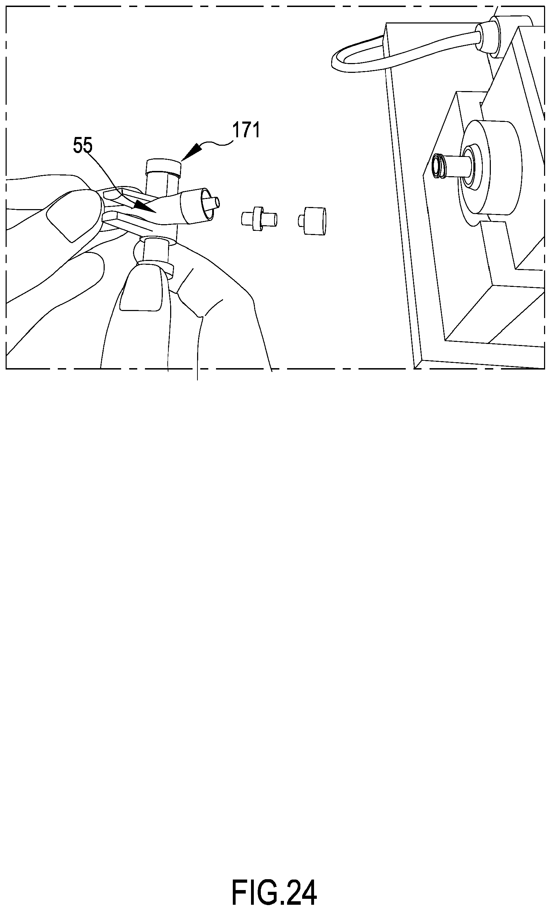

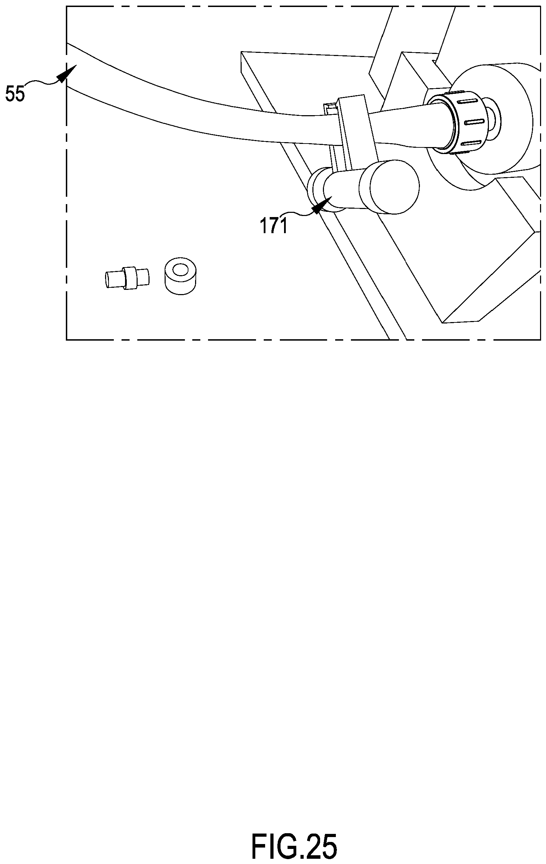

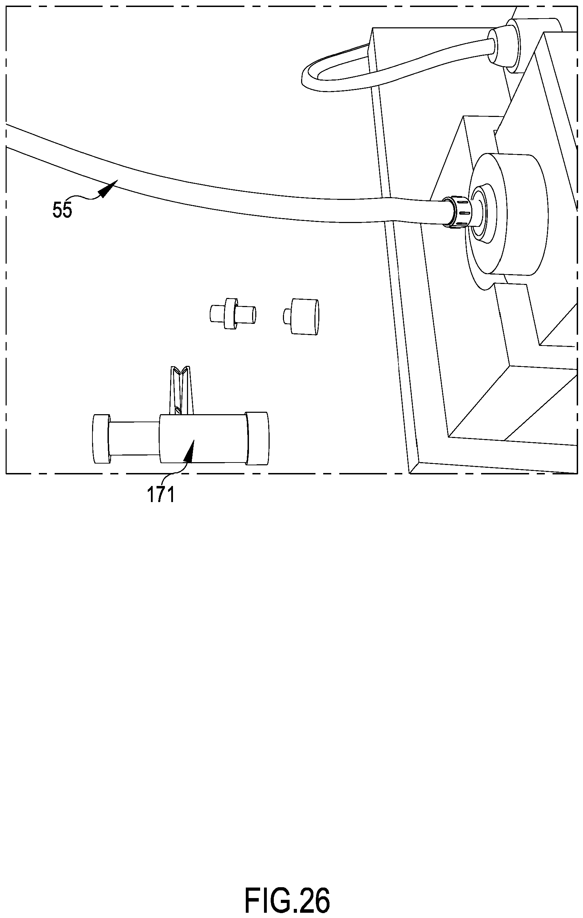

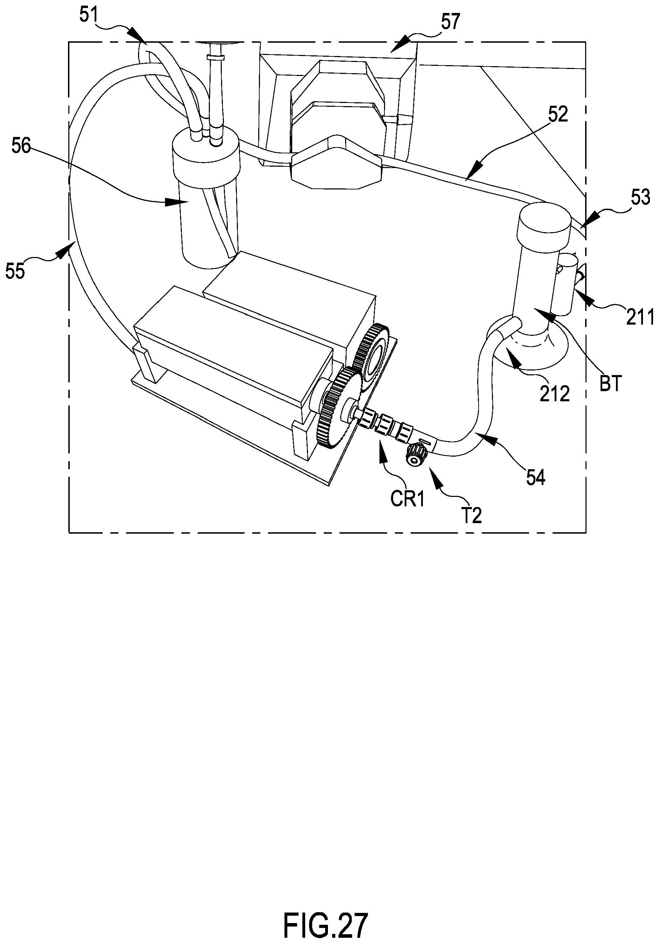

The connection method is based on the following seguential steps, subseguent to seeding (method for seeding a cell culture in the lumen of a scaffold according to the embodiment described above under point (1)) and at 24 hours from adhesion of the endothelial cells:
3.1) Connect - under sterility conditions - the tubes of the perfusion circuit (Fig. 22; 51 ; 52; 53; 54; 55), the element for removing the air bubbles (Fig. 22; BT), defined in the context of the present invention with the technical expression bubble trap, and the reservoir (Fig. 22; 56). The element for removing the air bubbles or bubble trap (Bt) consists of an element represented by a closed chamber, preferably made of glass, using a cap and having two asymmetric accesses: the access with the tap and connecting nozzle serves as an inflow (Fig. 27, 211) while access with the connecting nozzle only serves as an outflow (Fig. 27, 221). The bubble trap chamber contains a volume of liguid (hot fresh growth medium in this specific case) and a volume of air that traps possible air bubbles present in the perfusion liguid which flows through the two accesses of the bubble trap chamber.
3.2) Place the under-pump (Fig. 22; 52) of the closed perfusion circuit under the head of the peristaltic pump (Fig. 22; 57), which - upon activation - generates a peristaltic force capable of suctioning fluids, hot fresh growth medium in this case. The closed perfusion circuit is filled due to the suctioning, by the tube (Fig. 22; 51) of the perfusion circuit connected to the reservoir (Fig. 22; 56), of the hot fresh growth medium which is previously poured into the reservoir (Fig. 22; 56) in turn closed. Fill all the tubes of the perfusion circuit, the bubble trap (Fig. 22, BT) and the reservoir (Fig. 22; 56) with a liguid perfusion, such as a hot fresh growth medium (as defined above), until the hot fresh growth medium returns to the reservoir through the tube 55 (Fig. 22; 55) of the closed perfusion circuit. The BT and the reservoir are filled so as to leave a given air volume. In particular, the bubble trap (Fig. 22, BT) is filled so that said bubble trap chamber has a first part of the volume thereof filled with said fresh growth medium and a second part of the volume thereof filled with air, said second part of said volume having the function of trapping the air bubbles present in the perfusion liguid (hot fresh growth medium) which flows through said access serving as an inflow (211) and said access serving as an outflow (212) of the bubble trap (BT) (Fig. 22).
3.3) Occlude the tube 54 (Fig 23), preferably using a clamp (Fig. 23; 172) in a position proximal to the BT and move the tap of the BT to the closing position (in perpendicular position with respect to the tubes of the perfusion circuit).
3.4) Place the bioreactor-scaffold system under the same sterility conditions as the perfusion circuit. 3.5) Occlude the tube 55 (Fig. 23), preferably using a clamp (Fig. 23; 171; Fig. 17; 171) in a position proximal to the connection C with the tube 54 (Fig. 23; C). Open the upper and lateral ends of the T- shaped connector T2 arranged upstream of the bioreactor (Fig. 4; T2).
3.6) Upon removing the caps from the upper and lateral ends of the T-shaped connector T2 (Fig. 4) arranged upstream of the bioreactor, the possible creation of air bubbles is compensated by manually adding (preferably using a pasteur pipette) having the same volume as the hot fresh growth medium (Fig. 16).
3.7) Keep the tube 54 (Fig. 23) of the closed perfusion circuit in vertical position, unscrew the connector arranged between the tube 54 (Fig. 23) and the tube 55 (Fig. 23) of the perfusion circuit and preferably cap the tube 55 of the perfusion circuit using a cap (Fig. 24).
3.8) Screw the tube 54 (Fig. 27) of the perfusion circuit to the open lateral end of the T-shaped connector T2 (Fig. 27) upstream of the bioreactor at a lateral access thereof. The connector upstream of the bioreactor must always be kept in vertical position (Fig. 18 or 27).
3.9) Open the T-shaped connector T3 (Fig. 4) downstream of the bioreactor by unscrewing the cap of the lateral opening.
3.10) Unscrew the rotary connector CR2 together with the T-shaped connector T3 (Fig. 4), holding the toothed wheel R (Fig. 4) still and screw the connector of the tube 55 (Fig. 25) on the lateral opening of the scaffold-holder 14a (Fig. 1) (Fig. 25).
3.11) Remove the clamp 171 which occludes the tube 55 of the perfusion circuit (Fig. 26).
3.12) Position the bioreactor-seeded scaffold system connected to the perfusion circuit at about 37°C and at about 5% of CO2, place the under-pump 52 (Fig. 27) in free position, open the tap of the bubble trap BT (Fig. 27) by positioning it parallel to the tubes of the perfusion circuit, remove the clamp 172 (Fig. 27) and then start the pump (Fig. 27, 57). The peristaltic force applied by the pump to the assembled system, consisting of the perfusion circuit and the bioreactor-scaffold system will allow the perfusion of the scaffold.

Advantageously, the connection method (perfusion method) described herein, both in the first embodiment and in the second embodiment described above, allows to connect a perfusion circuit of a scaffold, preferably tubular, to the bioreactor-seeded scaffold system. This procedure allows to prevent the formation of air bubbles and prevents the bubbles, should they be formed, from reaching the scaffold seeded with endothelial cells of the bioreactor-scaffold system. Furthermore, the air bubbles possibly already present in the perfusion circuit do not reach the scaffold due to the presence of the bubble trap (Fig. 21, 71; Fig. 27, BT) in the circuit. In this manner, the method created by the Applicant is capable of ensuring complete absence of air bubbles in the lumen of the scaffold. This system meets the requirements set forth by the configuration of the bioreactor. All details indicated and described are required to make the method independent from the operator and thus for ensuring the reproducibility of the results during the in vitro generation of a construct with functional endothelium at industrial level. In addition, this method allows to be able to operate under sterility conditions, being based on simple actions. Furthermore, the quick and traceable procedure allows to reduce the risk that the air bubbles come into contact with the cells, thus avoiding damaging the endothelial cells adhered on the lumen of the scaffold, preferably tubular. Said perfusion method allows to obtain engineered vascular constructs/tissues having a scaffold having a lumen covered with a continuous (i.e. having a monolayer of confluent cells) and functional endothelium.

### Experimental evidence of the connection method

The experimental analysis regarding the evaluation of the method for connecting the perfusion circuit to the bioreactor-scaffold system are the same ones applied for the evaluation of the seeding method of a cell culture in a scaffold preferably tubular.

In the context of the present invention the perfusion circuit (Fig. 5 and Fig. 22) is defined as an assembly of: tubes (Fig. 5; 51- 54 or Fig. 22; 51-55), a reservoir (Fig. 5 or Fig. 22; 56), a peristaltic pump (Fig. 5; 55 or Fig. 22; 57) and an element for removing the air bubbles (Fig. 21, 71 or Fig. 22, BT). Said element for removing the air bubbles can be present in the perfusion circuit prior to the connection of the perfusion circuit to the bioreactor-seeded scaffold system (second embodiment of the perfusion method) or, alternatively, it may be inserted into the perfusion circuit after the connection of the perfusion circuit to the bioreactor-seeded scaffold system (first embodiment of the perfusion method). Said tubes are made of biocompatible material and are connected to each other so as to allow the perfusion of the scaffold (Fig. 21 and Fig. 27), preferably tubular, housed in the bioreactor 11 , by means of the peristaltic pump (Fig. 5; 55, Fig. 22; 57) (in such case, Masterflex^{®}, L/S Digital Dispensing Pump Drives 07551-20, Cole-Parmer) with the Easy-Load II 77200-62 (Masterflex, Cole-Parmer) head. With reference to then second embodiment of the perfusion method described above and illustrated in figure 27, the perfusion circuit mainly consists of five tubes with an inner diameter of 3/16": a first tube 51 for suctioning from the reservoir, a second under-pump tube 52, a third tube 53 which connects the circuit to the bubble trap BT, a fourth tube 54 which connects the BT to the T-shaped connector T2 upstream of the bioreactor-scaffold system, a fifth tube 55 for return to the reservoir 56 connected to the T-shaped connector T3 downstream of the bioreactor-scaffold system.

With reference to the first embodiment of the perfusion method described above and represented in figure 5, the tube 51 is connected to the under-pump tube 52, the under-pump tube 52 to the BT, the BT to the tube 53, the tube 53 to the T-shaped connector T2 upstream of the bioreactor-scaffold system, the tube 54 to the T-shaped connector T3 downstream of the bioreactor-scaffold system and to the reservoir 56. The reservoir (Fig. 5 or Fig. 22, 56) is the element containing the hot fresh growth medium (for example Endothelial Growth Medium EGM, Sigma Aldrich) from which the tube 51 (Fig. 5 or Fig. 22) suctions and to which the tube 54 (Fig. 5) or 55 (Fig. 22) returns, keeping the entire bioreactor-seeded scaffold system in a closed system. The reservoir (Fig. 5 or Fig. 22, 56) is under atmospheric pressure, due to a 0.22 m filter present in the cap of the reservoir, which guarantees the sterility of the air.

The first phase to be carried out and optimised is the cell seeding phase, preferably endothelial cells, followed by a second critical phase of connecting the perfusion circuit to the system comprising the bioreactor and the scaffold, so as to ensure reliability, effectiveness and reproducibility to the industrialisation process for the in vitro generation of a continuous and functional endothelium.

The success of the industrialisation process subject of the present invention for the production of the engineered vascular tissue/construct, as claimed mainly consists in the success relating to the phase of seeding and connecting the perfusion circuit to the bioreactor-scaffold system, so as to globally guarantee the elimination of air bubbles in a reliable and reproducible manner. The seeding method depends on the cell source and on the density thereof, on the chemical and porosity properties and on the full removal of the air bubbles from the lumen of the scaffold during the injection of the cell suspension. On the other hand, the method for connection between the perfusion circuit and the bioreactor-scaffold system is based on maintaining the sterility of the assembled system and on the guarantee of absence of air bubbles that can come into contact with the seeded scaffold. It should be observed that the formation of air bubbles must be avoided given that the air bubbles can damage the cells, jeopardising the viability thereof with ensuing lack of endothelisation of the scaffolds.

The description of the present invention shows that the choice of the method for connecting the perfusion circuit to the bioreactor-scaffold system depends on the method for seeding the previously optimised endothelial cells due to the fact that said connection method must be suitable to the experimental setup and to the perfusion needs and the position chosen for this system in the incubator. The process for seeding a scaffold, preferably a tubular scaffold, is one of the factors crucial towards in vitro generation of functional engineered vascular constructs, using confluent endothelium, as shown in the description of the present invention. This process is responsible for a uniform and homogeneous distribution of endothelial cells in the lumen same case applying to the adhesion of the cells to the surface. The description of the present invention shows that the choice of the most appropriate seeding method, adapting it to each bioreactor-scaffold system, defines the reproducibility of the process, showing the advantages thereof in the reproducibility of the results.

Also in preclinical tests, as well as others, there arises the need for creating reproducible methods for the large-scale production of functional vascular constructs.

Thus, the seeding method of the present invention, well defined and traceable, guarantees a highly uniform distribution in terms of adhesion of endothelial cells and good reproducibility of the results, required for a laboratory whose activity focuses on the production of in vitro vascular constructs as test models of the preclinical field as well as other fields.

After 24 hours of adhesion, the cells adhered in the lumen of the scaffold, mainly tubular, must maintain their morphology and viability so as to obtain a homogeneous vascular endothelium. Thus, it is important to avoid any cell alteration during the connection process which could alter the state of cell adhesion, with possible loss of the vascular layer subject of growth (being formed).

The known methods for connection between the bioreactor and the perfusion circuit cause cell suffering with ensuing detachment - even partial - of the endothelial cells from the luminal surface, so that the formation of a functional endothelial layer is slowed or hindered. Furthermore, the known methods for connection between the bioreactor and the perfusion circuit do not guarantee the absence of air bubbles, that may be formed due to possible torsions or compressions (full or partial) of the connection tubes during the perfusion. It is important to absolutely prevent the contact between said air bubbles and the seeded scaffold which is avoided by introducing an element, for example a bubble-trap capable of eliminating the air bubbles before they reach the seeded scaffold. This drawback was successfully overcome thanks to the process subject of the present invention which allows to obtain an inner surface of the scaffold covered with a uniform and functional layer of endothelial cells, in particular a confluent cell layer).

### LIST OF REFERENCE NUMBERS

11 bioreactor
13 scaffold-holder
13a scaffold-holder grip
13b scaffold-holder grip
14a lateral opening of the scaffold-holder
21 scaffold
41 inflow of the bioreactor chamber
42 outflow of the bioreactor chamber
51 tube
52 tube or under-pump
53 tube
54 tube
55 head of the peristaltic pump
56 reservoir
57 pump
71 element for removing air bubbles (or bubble trap)
72 cap
91 container, preferably syringe
101 pipette
102 syringe plunger
171 clamp
172 clamp
211 access with inflow function
212 access with outflow function
BT bubble trap
CR1 rotary connector
CR2 rotary connector
T1 T-shaped connector
T2 T-shaped connector
T3 T-shaped connector

## Claims

1. An in-vitro model of a tubular-shaped human vascular structure having dysfunctional anatomical and physiological characteristics simulating the same vascular structure of a healthy subject whose vascular structure has been damaged or deformed or deteriorated due to a damage selected from among the group comprising or, alternatively, consisting of aneurysm, stenosis, sclerosis plaques, forms of tumours or cardiomyopathies; wherein said model comprises or, alternatively, consists of one or more biocompatible porous polymeric supports scaffold(21) capable of promoting a cell adhesion and growth, wherein said scaffold has been seeded with endothelial cells which cover a lumen of the scaffold and constitute an endothelium having a single layer of confluent cells, said scaffold (21) has been made with deformities or defects on a tubular structure thereof, said deformities or defects comprising curvatures, elbows, constrictions, dilatations; said scaffold (21) consisting of electrospun silk fibroin, copolymers of polyglycolic, acid/polylactic acid (PGA/PLA) or copolymers of polyglycolic acid/polycaprolactone (PGA/PCL).

2. The in vitro model according to claim 1, wherein said vascular structure is selected from among blood vessels or blood ducts.

3. The in vitro model according to claim 1 or 2, wherein said vascular structure is selected from among arteries and veins.

4. The in vitro model according to any one of claims 1-3, wherein said deformities or said defects of the tubular structure comprise combinations of, curvatures, elbows, constrictions, dilatations.

5. The *in vitro* model according to any one of claims 1-4, wherein the endothelial cells are selected from among the cells that form an endothelium of vascular tissue.

6. The in vitro model according to claim 5, wherein the endothelial cells are selected from HAOECs (*human aortic endothelial cells*)*,* HCAECs (*human coronary endothelial cells*), HMEVECs (*human dermal microvascular endothelial cells*) or HUVECs (*human umbilical vein endothelial cells*)*.*

7. A method for testing a medical device or a drug so as to verify the effectiveness and safety thereof before an in-vivo use thereof on the man, said method comprising the following steps:
preparing a tubular-shaped scaffold (21) having the dysfunctional anatomical and physiological characteristics suitable to simulate a damage or a deformation or a deterioration due to an aneurysm, stenosis, sclerosis plaques, forms of tumours or cardiomyopathies, said scaffold (21) having deformities or defects on the tubular structure thereof which are curvatures, elbows, constrictions, dilatations; said scaffold (21) consisting of electrospun silk fibroin, copolymers of polyglycolic acid/polylactic acid (PGA/PLA) or copolymers of polyglycolic acid/ polycaprolactone (PGA/PCL);
seeding at least one part of the interior lumen of said scaffold (21) with endothelial cell lines so as to obtain a continuous and homogeneous layer of seeded endothelial cells (seeding method), optionally seeding at least one part of the outer surface of said scaffold (21) with muscle cell lines;
promoting the growth of said endothelial cells, and optionally said muscle cells, up to obtaining a single continuous and
uniform layer of endothelial cells, to obtain said in vitro model;
introducing into said in vitro model a medical device or a drug subject of test, and
allowing the circulation (perfusion model) in said in-vitro model comprising said medical device or drug of a human whole blood sample, artificial blood or derivatives thereof so as to evaluate the behaviour and the interaction of said medical device or drug with said human whole blood sample, artificial blood or derivatives thereof.

8. A method or process for the production of an engineered vascular tissue or construct that is the scaffold (21) in the in vitro model according to any one of claims 1-6, for testing medical products, said process comprising applying:
- a method for seeding an endothelial cell culture in the lumen of said scaffold (21) to obtain a seeded scaffold (21); said seeded scaffold (21) being present in a bioreactor (11), to obtain a bioreactor (11)- seeded scaffold (21) system;
wherein said seeding method comprises the steps of:
- releasing said endothelial cell culture in form of a cell suspension comprising a fresh growth medium and endothelial cells in a container (91) mounted on a T-shaped connector (T2) arranged upstream of the bioreactor (11) by means of a rotary connector (CR1); followed by
- releasing said endothelial cell culture in the lumen of the scaffold (21) present in the bioreactor chamber (11) with a continuous flow such that the flow speed allows said cell suspension to drip into the T-shaped connector (T2) without generating air bubbles and pushing the air bubbles present in the lumen of the scaffold (21) towards an opening of a T-shaped connector (T3) arranged downstream of the bioreactor (11) allowing the outflow thereof;
and, subsequently,
- a method for perfusion - with a fresh growth medium having a temperature comprised in the range between 30°C and 45°C, preferably at 37°C - of the endothelial cells present in the lumen of said seeded scaffold (21); said perfusion method being obtained by connecting a perfusion circuit (51-56) or (51-57 and BT) to said bioreactor (11)-seeded scaffold (21) system;
wherein said perfusion method comprises a step of
- partly filling an element for removing the air bubbles (71) or (BT) present in the perfusion circuit with said fresh growth medium, wherein said element for removing the air bubbles (71) or (BT) comprises a chamber, a cap that closes said chamber, an access with inflow function (211) and an access with outflow function (212), wherein said chamber of the element for removing the air bubbles (71 or BT) has a volume and wherein a first part of said volume is filled with said fresh growth medium and wherein a second part of said volume is filled with air, said second part of said volume having the function of trapping the air bubbles present in said fresh growth medium which flows through said access with inflow function (211) and said access with outflow function (212).

9. The process according to claim 7 or 8, wherein said method for seeding said endothelial cell culture in the lumen of said scaffold (21) comprises:
- mounting the scaffold (21) on the grips of a scaffold-holder (13, 13a, 13b) and housing said scaffold- holder (13, 13a, 13b) with the scaffold (21) in the bioreactor chamber (11), to obtain a bioreactor(11)- scaffold (21) system;
followed by
- injecting the fresh growth medium into the lumen of said scaffold (21) fixed on said scaffold-holder (13) arranged inside the bioreactor chamber (11); followed by
- adding said fresh growth medium into the bioreactor chamber (11) where said scaffold-holder (13, 13a, 13b) with the scaffold (21) is present injected with said growth medium; followed by
- leaving for a time interval comprised between 1 hour and 18 hours at a temperature comprised between 20°C and 30°C, preferably 25°C, said growth medium in the lumen of the scaffold (21) and in the bioreactor chamber (11) where said scaffold-holder (13) with the scaffold (21) is present injected with said growth medium; followed by clearing the internal of the lumen of the scaffold (21) and of the bioreactor chamber (11) of the growth medium; followed by
- releasing said endothelial cell culture in said container (91), preferably said container (91) being a syringe; followed by
- releasing said cell suspension in the lumen of the scaffold (21) according to claim 1; followed by
- adding said fresh growth medium in the bioreactor chamber (11) where said scaffold-holder (13) with the scaffold (21) is present seeded containing said cell suspension in the lumen; and followed by
- incubating, preferably for 24 hours at 37°C in presence of 5% of C02, the scaffold (21) housed in the bioreactor chamber (11).

10. The process according to any one of claims 7-9, wherein said method for the perfusion of the endothelial cells present in the lumen of said seeded scaffold (21) comprises:
preparing said closed perfusion circuit comprising the tubes (51), (52), (53), (54), and, optionally, (55);
- occluding the tube (54) or (55) of the perfusion circuit using a closing element (171) in a position proximal to a connector (C), preferably said closing element is a clamp or the like; followed by
- unscrewing the connector (C) arranged between the tube (53) or (54) and the tube (54) or (55) respectively in the perfusion circuit;
- screwing the tube (53) or (54) of the perfusion circuit to an open lateral end of the T-shaped connector (T2) upstream of the bioreactor (11) at a lateral access thereof; followed by
- opening the T-shaped connector (T3) downstream of the bioreactor (11) and unscrewing a cap of a lateral opening of the T-shaped connector (T3); followed by
- connecting the tube (54) or (55) of the perfusion circuit to the lateral opening of the T-shaped connector (T3) arranged downstream of the bioreactor (11) and removing the closing element (171);
followed, if need be, by
- inserting - between the tube (53) and the under-pump tube (52) of the perfusion circuit - the element for removing the air bubbles (71).

11. The process according to any one of the preceding claims 7-10, wherein the element for removing the air bubbles (71) or (BT) is a *bubble-trap* or the like.

12. The process according to any one of the preceding claims 8-11, wherein the growth medium used is the Endothelial Growth Medium comprising fetal bovine serum (2%), adenine (0.2 µg/ml), ammonium metavanadate (0.0006 µg/ml), amphotericin B (0.3 µg/ml), calcium chloride 2H20(300 µg/ml), choline chloride (20 µg/ml), copper sulphate 5H20 (0.002 µg/ml), trioptic acid DL-6,8(0.003 µg/ml), folinic acid (calcium) (0.6 µg/ml), heparin (4 µg/ml), hydrocortisone (2 µg/ml), L-aspartic acid (15 µg/ml), L-cysteine (30 µg/ml), L-tyrosine (20 µg/ml), manganese sulphate monohydrate (0.0002 µg/ml), ammonium molybdate 4H20 (0.004 µg/ml), nicotinamide (8 µg/ml), nickel chloride 6H20 (0.0001 µg/ml), penicillin (60 µg/ml), phenol red sodium salt (15 µg/ml), potassium chloride (300 µg/ml), putrescine dihydrochloride (0.0002 µg/ml), pyridoxine hydrochloride (3 µg/ml), sodium metasilicate 9H2O (3 µg/ml), sodium sulphate 7H2O (200 µg/ml), sodium selenite (0.01 µg/ml), streptomycin sulphate (100 µg/ml), thiamine hydrochloride (4 µg/ml) and zinc sulphate 7H2O (0.0003 µg/ml), preferably heated to 37°C.

13. A scaffold (21) for use in a preparation of an in vitro model according to claims 1-6, having a lumen coated with a functional and continuous endothelium (21) having a confluent cell monolayer obtained by means of a process comprising the following steps:
- preparing a tubular-shaped scaffold having the dysfunctional anatomical and physiological characteristics suitable to simulate a damage or a deformation or a deterioration due to an aneurysm, stenosis, sclerosis plaques, forms of tumours or cardiomyopathies, wherein the scaffold is being made with deformities or defects on a tubular structure thereof, said deformities or defects comprising curvatures, elbows, constrictions, dilatations and consists of electrospun silk fibroin, copolymers of polyglycolic acid/polylactic acid (PGA/PLA) or copolymers of polyglycolic acid/polycaprolactone (PGA/PCL);
- seeding at least one part of the interior lumen of said scaffold with endothelial cell lines so as to obtain a continuous and homogeneous layer of seeded endothelial cells (seeding method), optionally seeding at least one part of the outer surface of said scaffold with muscle cell lines;
- promoting the growth of said endothelial cells, and optionally said muscle cells, up to obtaining a continuous and uniform layer of endothelial cells, to obtain said in vitro model.

14. Use of the scaffold (21) according to any one of claims 1-6 or 13, for conducting in vitro preclinical or clinical tests of a medicinal product for human use to be used in the cardiovascular and peripheral vascular region.

## Patentansprüche

1. In-vitro-Modell einer röhrenförmigen menschlichen Gefäßstruktur mit dysfunktionalen anatomischen und physiologischen Eigenschaften, die die gleiche Gefäßstruktur eines gesunden Probanden simulieren, dessen Gefäßstruktur aufgrund einer Schädigung, die aus der Gruppe ausgewählt ist, die Aneurysma, Stenose, Sklerose-Plaques, Formen von Tumoren oder Kardiomyopathien umfasst oder alternativ daraus besteht, beschädigt oder deformiert oder verschlechtert wurde; wobei das Modell ein oder mehrere biokompatible poröse polymere Trägergerüste (21) umfasst oder alternativ daraus besteht, die in der Lage sind, eine Zelladhäsion und ein Zellwachstum zu fördern, wobei das Gerüst mit Endothelzellen ausgesät wurde, die ein Lumen des Gerüsts bedecken und ein Endothel mit einer einzigen Schicht konfluenter Zellen bilden, wobei das Gerüst (21) mit Deformitäten oder Defekten an einer röhrenförmigen Struktur davon hergestellt wurde, wobei die Deformitäten oder Defekte Krümmungen, Knickstellen, Verengungen, Dilatationen umfassen; wobei das Gerüst (21) aus elektrogesponnenem Seidenfibroin, Copolymeren von Polyglykolsäure/Polymilchsäure (PGA/PLA) oder Copolymeren von Polyglykolsäure/Polycaprolacton (PGA/PCL) besteht.

2. In-vitro-Modell nach Anspruch 1, wobei die Gefäßstruktur aus Blutgefäßen oder Blutkanälen ausgewählt ist.

3. In-vitro-Modell nach Anspruch 1 oder 2, wobei die Gefäßstruktur aus Arterien und Venen ausgewählt ist.

4. In-vitro-Modell nach einem der Ansprüche 1-3, wobei die Deformitäten oder Defekte der röhrenförmigen Struktur Kombinationen von Krümmungen, Knickstellen, Verengungen, Dilatationen umfassen.

5. In-vitro-Modell nach einem der Ansprüche 1-4, wobei die Endothelzellen aus den Zellen ausgewählt sind, die ein Endothel von Gefäßgewebe bilden.

6. In-vitro-Modell nach Anspruch 5, wobei die Endothelzellen ausgewählt sind aus HAOECs (humane Aortenendothelzellen), HCAECs (*humane Koronararterien-Endothelzellen*), HMEVECs (*humane dermale mikrovaskuläre Endothelzellen*) oder HUVECs (*humane Nabelvenen-Endothelzellen) .*

7. Verfahren zum Testen einer medizinischen Vorrichtung oder eines Arzneimittels, um deren Wirksamkeit und Sicherheit vor einer In-vivo-Verwendung am Menschen zu überprüfen, wobei das Verfahren die folgenden Schritte umfasst:
Vorbereiten eines röhrenförmigen Gerüsts (21) mit den dysfunktionalen anatomischen und physiologischen Eigenschaften, die geeignet sind, eine Schädigung oder eine Verformung oder eine Verschlechterung aufgrund eines Aneurysmas, Stenose, Sklerose-Plaques, Formen von Tumoren oder Kardiomyopathien zu simulieren, wobei das Gerüst (21) Deformitäten oder Defekte an seiner röhrenförmigen Struktur aufweist, die Krümmungen, Knickstellen, Verengungen, Dilatationen sind; wobei das Gerüst (21) aus elektrogesponnenem Seidenfibroin, Copolymeren von Polyglykolsäure/Polymilchsäure (PGA/PLA) oder Copolymeren von Polyglykolsäure/ Polycaprolacton (PGA/PCL) besteht;
Aussäen mindestens eines Teils des Innenlumens des Gerüsts (21) mit Endothelzelllinien, um eine kontinuierliche und homogene Schicht von ausgesäten Endothelzellen zu erhalten (Aussaatverfahren), optional Aussäen mindestens eines Teils der Außenfläche des Gerüsts (21) mit Muskelzelllinien;
Fördern des Wachstums der Endothelzellen und optional der Muskelzellen bis zum Erhalt einer einzigen kontinuierlichen und gleichmäßigen Schicht von Endothelzellen, um das In-vitro-Modell zu erhalten;
Einführen einer medizinischen Vorrichtung oder eines Arzneimittels, die/das zu testen ist, in das In-vitro-Modell, und
Ermöglichen der Zirkulation (Perfusionsmodell) in dem In-vitro-Modell, das die medizinische Vorrichtung oder Arzneimittel einer menschlichen Vollblutprobe, künstlichen Bluts oder Derivate davon umfasst, um das Verhalten und die Wechselwirkung der medizinischen Vorrichtung oder des Arzneimittels mit der menschlichen Vollblutprobe, künstlichem Blut oder Derivate davon zu bewerten.

8. Verfahren oder Prozess zur Herstellung eines entwickelten Gefäßgewebes oder -konstrukts, das das Gerüst (21) in dem In-vitro-Modell nach einem der Ansprüche 1-6 ist, zum Testen von medizinischen Produkten, wobei der Prozess Folgendes umfasst:
- ein Verfahren zum Aussäen einer Endothelzellkultur im Lumen des Gerüsts (21), um ein ausgesätes Gerüst (21) zu erhalten; wobei das ausgesäte Gerüst (21) in einem Bioreaktor (11) vorhanden ist, um ein System aus Bioreaktor (11)-ausgesätem Gerüst (21) zu erhalten;
wobei das Aussaatverfahren die folgenden Schritte umfasst:
- Freisetzen der Endothelzellkultur in Form einer Zellsuspension, die ein frisches Wachstumsmedium und Endothelzellen in einem Behälter (91) umfasst, der auf einem T-förmigen Verbinder (T2) montiert ist, der stromaufwärts des Bioreaktors (11) mittels eines Drehverbinders (CR1) angeordnet ist; gefolgt von
- Freisetzen der Endothelzellkultur in das Lumen des in der Bioreaktorkammer (11) vorhandenen Gerüsts (21) mit einem kontinuierlichen Strom, so dass die Strömungsgeschwindigkeit es der Zellsuspension ermöglicht, in den T-förmigen Verbinder (T2) zu tropfen, ohne Luftblasen zu erzeugen und die im Lumen des Gerüsts (21) vorhandenen Luftblasen in Richtung einer Öffnung eines T-förmigen Verbinders (T3) zu drücken, der stromabwärts des Bioreaktors (11) angeordnet ist, indem dessen Ausströmen ermöglicht wird;
und anschließend
- ein Verfahren zur Perfusion - mit einem frischen Wachstumsmedium mit einer Temperatur im Bereich zwischen 30 °C und 45 °C, vorzugsweise bei 37 °C - der im Lumen des ausgesäten Gerüsts (21) vorhandenen Endothelzellen; wobei das Perfusionsverfahren durch Verbinden eines Perfusionskreislaufs (51-56) oder (51-57 und BT) mit dem System aus Bioreaktor (11)-ausgesätem Gerüst (21) erhalten wird;
wobei das Perfusionsverfahren einen Schritt umfasst, zum
- teilweisen Befüllen eines Elements zum Entfernen der in dem Perfusionskreislauf vorhandenen Luftblasen (71) oder (BT) mit dem frischen Wachstumsmedium, wobei das Element zum Entfernen der Luftblasen (71) oder (BT) eine Kammer, eine die Kammer verschließende Kappe, einen Zugang mit Einströmfunktion (211) und einen Zugang mit Ausströmfunktion (212) umfasst, wobei die Kammer des Elements zum Entfernen der Luftblasen (71 oder BT) ein Volumen aufweist und wobei ein erster Teil des Volumens mit dem frischen Wachstumsmedium gefüllt ist und wobei ein zweiter Teil des Volumens mit Luft gefüllt ist, wobei der zweite Teil des Volumens die Funktion hat, die Luftblasen einzufangen, die in dem frischen Wachstumsmedium vorhanden sind, das durch den Zugang mit Einströmfunktion (211) und den Zugang mit Ausströmfunktion (212) strömt.

9. Prozess nach Anspruch 7 oder 8, wobei das Verfahren zum Aussäen der Endothelzellkultur im Lumen des Gerüsts (21) umfasst:
- Montieren des Gerüsts (21) an den Griffen eines Gerüsthalters (13, 13a, 13b) und Unterbringen des Gerüsthalters (13, 13a, 13b) mit dem Gerüst (21) in der Bioreaktorkammer (11), um ein System aus Bioreaktor (11)-Gerüst (21) zu erhalten;
gefolgt von
- Injizieren des frischen Wachstumsmediums in das Lumen des Gerüsts (21), das an dem Gerüsthalter (13) befestigt ist, der innerhalb der Bioreaktorkammer (11) angeordnet ist; gefolgt von
- Zugeben des frischen Wachstumsmediums in die Bioreaktorkammer (11), in der der Gerüsthalter (13, 13a, 13b) mit dem Gerüst (21) vorhanden ist, das mit dem Wachstumsmedium injiziert wurde; gefolgt von
- Belassen für einen Zeitraum im Bereich zwischen 1 Stunde und 18 Stunden bei einer Temperatur im Bereich zwischen 20 °C und 30 °C, vorzugsweise 25 °C des Wachstumsmediums im Lumen des Gerüsts (21) und in der Bioreaktorkammer (11), in der der Gerüsthalter (13) mit dem Gerüst (21) vorhanden ist, das mit dem Wachstumsmedium injiziert wurde; gefolgt von
- Räumen des Inneren des Lumens des Gerüsts (21) und der Bioreaktorkammer (11) von dem Wachstumsmedium; gefolgt von
- Freisetzen der Endothelzellkultur in den Behälter (91), wobei vorzugsweise der Behälter (91) eine Spritze ist; gefolgt von
- Freisetzen der Zellsuspension in das Lumen des Gerüstes (21) nach Anspruch 1; gefolgt von
- Zugeben des frischen Wachstumsmediums in die Bioreaktorkammer (11), in der der Gerüsthalter (13) mit dem Gerüst (21) vorhanden ist, der ausgesät ist und die Zellsuspension im Lumen enthält; und gefolgt von
- Inkubieren des Gerüsts (21), das in der Bioreaktorkammer (11) untergebracht ist, vorzugsweise für 24 Stunden bei 37 °C in Gegenwart von 5 % CO2.

10. Prozess nach einem der Ansprüche 7-9, wobei das Verfahren zur Perfusion der Endothelzellen, die im Lumen des ausgesäten Gerüsts (21) vorhanden sind, umfasst:
Vorbereiten des geschlossenen Perfusionskreislaufs, der Schläuche (51), (52), (53), (54) umfasst und optional (55),
- Verschließen des Schlauchs (54) oder (55) des Perfusionskreislaufs unter Nutzung eines Verschlusselements (171) in einer Position proximal zu einem Verbinder (C), vorzugsweise ist das Verschlusselement eine Klemme oder dergleichen; gefolgt von
- Abschrauben des Verbinders (C), der zwischen dem Schlauch (53) oder (54) und dem Schlauch (54) oder (55) jeweils im Perfusionskreislauf angeordnet ist;
- Verschrauben des Schlauches (53) oder (54) des Perfusionskreislaufs auf einem offenen seitlichen Ende des T-förmigen Verbinders (T2) stromaufwärts des Bioreaktors (11) an einem seitlichen Zugang desselben; gefolgt von
- Öffnen des T-förmigen Verbinders (T3) stromabwärts des Bioreaktors (11) und Abschrauben einer Kappe einer seitlichen Öffnung des T-förmigen Verbinders (T3); gefolgt von
- Verbinden des Schlauchs (54) oder (55) des Perfusionskreislaufs auf der seitlichen Öffnung des T-förmigen Verbinders (T3), der stromabwärts des Bioreaktors (11) angeordnet ist, und Entfernen des Verschlusselements (171);
ggf. gefolgt von
- Einführen - zwischen dem Schlauch (53) und dem Unterpumpenschlauch (52) des Perfusionskreislaufs - des Elements zum Entfernen der Luftblasen (71).

11. Prozess nach einem der vorhergehenden Ansprüche 7-10, wobei das Element zum Entfernen der Luftblasen (71) oder (BT) eine Blasenfalle oder dergleichen ist.

12. Prozess nach einem der vorhergehenden Ansprüche 8-11, wobei das verwendete Wachstumsmedium das endotheliale Wachstumsmedium ist, umfassend fötales Rinderserum (2%), Adenin (0,2 pg/ml), Ammoniummetavanadat (0,0006 pg/ml), Amphotericin B (0,3 pg/ml), Calciumchlorid 2H2O(300 pg/ml), Cholinchlorid (20 pg/ml), Kupfersulfat 5H2O (0,002 pg/ml), Trioptinsäure DL-6,8 (0,003 pg/ml), Folinsäure (Calcium) (0,6 pg/ml), Heparin (4 pg/ml), Hydrocortison (2 pg/ml), L-Asparaginsäure (15 pg/ml), L-Cystein (30 pg/ml), L-Tyrosin (20 pg/ml), Mangansulfat-Monohydrat (0,0002 pg/ml), Ammoniummolybdat 4H2O (0,004 pg/ml), Nicotinamid (8 pg/ml), Nickelchlorid 6H2O (0,0001 pg/ml), Penicillin (60 pg/ml), Phenolrot-Natriumsalz (15 pg/ml), Kaliumchlorid (300 pg/ml), Putrescindihydrochlorid (0,0002 pg/ml), Pyridoxinhydrochlorid (3 pg/ml), Natriummetasilikat 9H2O (3 pg/ml), Natriumsulfat 7H2O (200 pg/ml), Natriumselenit (0,01 pg/ml), Streptomycinsulfat (100 pg/ml), Thiaminhydrochlorid (4 pg/ml) und Zinksulfat 7H2O (0,0003 pg/ml), vorzugsweise auf 37 °C erwärmt.

13. Gerüst (21) zur Verwendung bei einer Vorbereitung eines In-vitro-Modells nach den Ansprüchen 1-6, aufweisend ein Lumen, das mit einem funktionellen und kontinuierlichen Endothel (21) beschichtet ist, das eine konfluente Zellmonoschicht aufweist, die mittels eines Prozesses erhalten wurde, das die folgenden Schritte umfasst:
- Vorbereiten eines röhrenförmigen Gerüsts mit den dysfunktionalen anatomischen und physiologischen Eigenschaften, die geeignet sind, eine Schädigung oder eine Verformung oder eine Verschlechterung aufgrund eines Aneurysmas, einer Stenose, Sklerose-Plaques, Formen von Tumoren oder Kardiomyopathien zu simulieren, wobei das Gerüst mit Deformitäten oder Defekten an einer röhrenförmigen Struktur davon hergestellt wird, wobei die Deformitäten oder Defekte Krümmungen, Knickstellen, Verengungen, Dilatationen umfassen, und aus elektrogesponnenem Seidenfibroin, Copolymeren von Polyglykolsäure/Polymilchsäure (PGA/PLA) oder Copolymeren von Polyglykolsäure/Polycaprolacton (PGA/PCL) besteht;
- Aussäen mindestens eines Teils des Innenlumens des Gerüsts mit Endothelzelllinien, um eine kontinuierliche und homogene Schicht von ausgesäten Endothelzellen zu erhalten (Aussaatverfahren), optional Aussäen mindestens eines Teils der Außenfläche des Gerüsts mit Muskelzelllinien;
- Fördern des Wachstums der Endothelzellen und optional der Muskelzellen bis zum Erhalt einer kontinuierlichen und gleichmäßigen Schicht von Endothelzellen, um das In-vitro-Modell zu erhalten.

14. Verwendung des Gerüsts (21) nach einem der Ansprüche 1-6 oder 13 zur Durchführung von präklinischen oder klinischen In-vitro-Tests eines Arzneimittels für den menschlichen Gebrauch, das im kardiovaskulären und peripheren Gefäßbereich zu verwenden ist.

## Revendications

1. Modèle *in vitro* d'une structure vasculaire humaine de forme tubulaire comportant des caractéristiques anatomiques et physiologiques dysfonctionnelles simulant la même structure vasculaire d'un sujet sain dont la structure vasculaire a été endommagée, ou déformée ou détériorée en raison d'une lésion choisie dans le groupe comprenant ou, alternativement, constitué par un anévrisme, une sténose, des plaques de sclérose, des formes de tumeurs ou des cardiomyopathies ;
dans lequel ledit modèle comprend ou, alternativement, est constitué d'un ou plusieurs échafaudages de supports polymères poreux biocompatibles (21) capables de favoriser l'adhésion et la croissance des cellules, dans lequel ledit échafaudage a été ensemencé avec des cellules endothéliales qui couvrent une lumière de l'échafaudage et constituent un endothélium comportant une seule couche de cellules confluentes, ledit échafaudage (21) a été fabriqué avec des déformations ou des défauts sur une structure tubulaire de celui-ci, lesdites déformations ou défauts comprenant des courbures, des coudes, des constrictions, des dilatations ; ledit échafaudage (21) est constitué de fibroïne de soie électrofilée, de copolymères d'acide polyglycolique/d'acide polylactique (PGA/PLA) ou de copolymères d'acide polyglycolique/polycaprolactone (PGA/PCL).

2. Modèle *in vitro* selon la revendication 1, dans lequel ladite structure vasculaire est choisie parmi les vaisseaux sanguins ou les canaux sanguins.

3. Modèle *in vitro* selon la revendication 1 ou 2, dans lequel ladite structure vasculaire est choisie parmi les artères et les veines.

4. Modèle *in vitro* selon l'une quelconque des revendications 1-3, dans lequel lesdites déformations ou lesdits défauts de la structure tubulaire comprennent des combinaisons de courbures, de coudes, de constrictions, de dilatations.

5. Modèle *in vitro* selon l'une quelconque des revendications 1-4, dans lequel les cellules endothéliales sont choisies parmi les cellules qui forment un endothélium d'un tissu vasculaire.

6. Modèle *in vitro* selon la revendication 5, dans lequel les cellules endothéliales sont choisies parmi les HAOEC (cellules endothéliales aortiques humaines), les HCAEC (cellules endothéliales d'artères coronaires humaines), les HMEVEC (cellules endothéliales microvasculaires de derme humain) ou les HUVEC (cellules endothéliales de la veine ombilicale humaine).

7. Procédé pour tester un dispositif médical ou un médicament afin d'en vérifier l'efficacité et la sécurité avant de l'utiliser *in vivo* sur l'homme, ledit procédé comprenant les étapes suivantes :
préparer un échafaudage (21) de forme tubulaire comportant des caractéristiques anatomiques et physiologiques dysfonctionnelles adaptées pour simuler une lésion ou une déformation ou une détérioration due à un anévrisme, une sténose, des plaques de sclérose, des formes de tumeurs ou des cardiomyopathies, ledit échafaudage (21) comportant des déformations ou des défauts sur sa structure tubulaire étant des courbures, des coudes, des constrictions, des dilatations ; ledit échafaudage (21) est constitué de fibroïne de soie électrofilée, de copolymères d'acide polyglycolique/d'acide polylactique (PGA/PLA) ou de copolymères d'acide polyglycolique/ polycaprolactone (PGA/PCL) ;
ensemencer au moins une partie de la lumière intérieure dudit échafaudage (21) avec des lignées cellulaires endothéliales de manière à obtenir une couche continue et homogène de cellules endothéliales ensemencées (procédé d'ensemencement), éventuellement ensemencer au moins une partie de la surface extérieure dudit échafaudage (21) avec des lignées cellulaires musculaires ;
favoriser la croissance desdites cellules endothéliales, et éventuellement desdites cellules musculaires, jusqu'à obtenir une seule couche continue et homogène de cellules endothéliales, pour obtenir ledit modèle *in vitro* ;
introduire dans ledit modèle *in vitro* un dispositif médical ou un médicament à tester, et
permettre la circulation (modèle de perfusion) dans ledit modèle *in vitro* comprenant ledit dispositif médical ou médicament d'un échantillon de sang complet humain, de sang artificiel ou de ses dérivés afin d'évaluer le comportement et l'interaction dudit dispositif médical ou médicament avec ledit échantillon de sang complet humain, de sang artificiel ou de ses dérivés.

8. Méthode ou procédé pour la production d'un tissu ou d'une construction vasculaire de synthèse qui est l'échafaudage (21) dans le modèle *in vitro* selon l'une quelconque des revendications 1-6, pour tester des produits médicaux, ledit procédé comprenant l'application :
- d'une méthode d'ensemencement d'une culture de cellules endothéliales dans la lumière dudit échafaudage (21) pour obtenir un échafaudage ensemencé (21) ; ledit échafaudage ensemencé (21) étant présent dans un bioréacteur (11), pour obtenir un système bioréacteur (11)-échafaudage ensemencé (21) ; dans lequel ladite méthode d'ensemencement comprend les étapes de :
- libérer ladite culture de cellules endothéliales sous forme d'une suspension cellulaire comprenant un milieu de culture frais et des cellules endothéliales dans un récipient (91) monté sur un connecteur en « T » (T2) disposé en amont du bioréacteur (11) au moyen d'un connecteur rotatif (CR1) ; puis
- libérer ladite culture de cellules endothéliales dans la lumière de l'échafaudage (21) présent dans la chambre du bioréacteur (11) avec un débit continu tel que la vitesse d'écoulement permette à ladite suspension cellulaire de s'égoutter dans le connecteur en « T » (T2) sans générer de bulles d'air et en poussant les bulles d'air présentes dans la lumière de l'échafaudage (21) vers une ouverture d'un connecteur en « T » (T3) disposé en aval du bioréacteur (11) permettant la sortie de ces dernières ;
et, ensuite,
- une méthode de perfusion, avec un milieu de culture frais ayant une température comprise entre 30 et 45 °C, de préférence à 37 °C, des cellules endothéliales présentes dans la lumière dudit échafaudage ensemencé (21) ; ladite méthode de perfusion est obtenue en raccordant un circuit de perfusion (51-56) ou (51-57 et BT) audit système bioréacteur (11)-échafaudage ensemencé (21) ;
dans lequel ladite méthode de perfusion comprend une étape de
- remplir partiellement un élément pour retirer les bulles d'air (71) ou (BT) présentes dans le circuit de perfusion avec ledit milieu de culture frais, dans lequel ledit élément de retrait des bulles d'air (71) ou (BT) comprend une chambre, un capuchon qui ferme ladite chambre, un accès avec fonction d'entrée (211) et un accès avec fonction de sortie (212), dans lequel ladite chambre de l'élément destiné à retirer les bulles d'air (71 ou BT) a un certain volume et dans lequel une première partie dudit volume est remplie avec ledit milieu de culture frais et dans lequel une deuxième partie dudit volume est remplie d'air, ladite deuxième partie dudit volume ayant la fonction de piéger les bulles d'air présentes dans ledit milieu de culture frais qui s'écoule à travers ledit accès à fonction d'entrée (211) et ledit accès à fonction de sortie (212).

9. Procédé selon la revendication 7 ou 8, dans lequel ladite méthode d'ensemencement de ladite culture de cellules endothéliales dans la lumière dudit échafaudage (21) comprend :
- monter l'échafaudage (21) sur les accrochages d'un porte-échafaudage (13, 13a, 13b) et loger ledit porte-échafaudage (13, 13a, 13b) avec l'échafaudage (21) dans la chambre du bioréacteur (11), pour obtenir un système bioréacteur (11)-échafaudage (21) ;
puis
- injecter le milieu de culture frais dans la lumière dudit échafaudage (21) fixé sur ledit porte-échafaudage (13) disposé à l'intérieur de la chambre du bioréacteur (11) ; puis
- ajouter ledit milieu de culture frais dans la chambre du bioréacteur (11) où se trouve ledit porte-échafaudage (13, 13a, 13b) avec l'échafaudage (21) injecté avec ledit milieu de culture ; puis
- laisser pendant un intervalle de temps compris entre 1 heure et 18 heures à une température comprise entre 20 C° et 30 °C, de préférence 25 °C, ledit milieu de culture dans la lumière de l'échafaudage (21) et dans la chambre du bioréacteur (11) où se trouve ledit porte-échafaudage (13) avec l'échafaudage (21) injecté avec ledit milieu de culture ; puis
- purifier l'intérieur de la lumière de l'échafaudage (21) et de la chambre du bioréacteur (11) du milieu de culture ; puis
- libérer ladite culture de cellules endothéliales dans ledit récipient (91), de préférence ledit récipient (91) étant une seringue ; puis
- libérer ladite suspension cellulaire dans la lumière de l'échafaudage (21) selon la revendication 1 ; puis
- ajouter ledit milieu de culture frais dans la chambre du bioréacteur (11) où se trouve ledit porte-échafaudage (13) avec l'échafaudage (21) ensemencé contenant ladite suspension cellulaire dans la lumière ; et puis
- incuber, de préférence pendant 24 heures à 37 °C en présence de 5 % de C02, l'échafaudage (21) logé dans la chambre du bioréacteur (11).

10. Procédé selon l'une quelconque des revendications 7-9, dans lequel ladite méthode de perfusion des cellules endothéliales présentes dans la lumière dudit échafaudage ensemencé (21) comprend :
préparer ledit circuit de perfusion fermé comprenant les tubes (51), (52), (53), (54) et, éventuellement, (55) ;
- obstruer le tube (54) ou (55) du circuit de perfusion à l'aide d'un élément de fermeture (171) dans une position proche d'un connecteur (C), de préférence ledit élément de fermeture est un collier ou similaire ; puis
- dévisser le connecteur (C) disposé entre le tube (53) ou (54) et le tube (54) ou (55) respectivement dans le circuit de perfusion ;
- visser le tube (53) ou (54) du circuit de perfusion à une extrémité latérale ouverte du connecteur en « T » (T2) en amont du bioréacteur (11) en correspondance d'un accès latéral de celui-ci ; puis
- ouvrir le connecteur en « T » (T3) en aval du bioréacteur (11) et dévisser un capuchon d'une ouverture latérale du connecteur en « T » (T3) ; puis
- raccorder le tube (54) ou (55) du circuit de perfusion à l'ouverture latérale du connecteur en « T » (T3) disposé en aval du bioréacteur (11) et retirer l'élément de fermeture (171) ;
puis, le cas échéant
- insérer, entre le tube (53) et le tube de sous-pompe (52) du circuit de perfusion, l'élément destiné à retirer les bulles d'air (71).

11. Procédé selon l'une quelconque des revendications précédentes 7-10, dans lequel l'élément permettant de retirer les bulles d'air (71) ou (BT) est un piège à bulles ou similaire.

12. Procédé selon l'une quelconque des revendications précédentes 8-11, dans lequel le milieu de culture utilisé est le milieu de culture endothélial comprenant du sérum bovin fœtal (2 %), de l'adénine (0,2 pg/ml), du métavanadate d'ammonium (0,0006 pg/ml), de l'amphotéricine B (0,3 pg/ml), du chlorure de calcium 2H2O (300 pg/ml), du chlorure de choline (20 pg/ml), du sulfate de cuivre 5H2O (0,002 pg/ml), de l'acide trioptique DL-6,8(0,003 pg/ml), de l'acide folinique (calcium) (0,6 pg/ml), de l'héparine (4 pg/ml), de l'hydrocortisone (2 pg/ml), de l'acide L-aspartique (15 pg/ml), de l'L-cystéine (30 pg/ml), de l'L-tyrosine (20 pg/ml), du sulfate de manganèse monohydraté (0,0002 pg/ml), du molybdate d'ammonium 4H2O (0,004 pg/ml), du nicotinamide (8 pg/ml), du chlorure de nickel 6H2O (0,0001 pg/ml), de la pénicilline (60 pg/ml), du sel de sodium de rouge de phénol (15 pg/ml), du chlorure de potassium (300 pg/ml), du dihydrochlorure de putrescine (0,0002 pg/ml), du chlorhydrate de pyridoxine (3 pg/ml), du métasilicate de sodium 9H2O (3 pg/ml), du sulfate de sodium 7H2O (200 pg/ml), du sélénite de sodium (0,01 pg/ml), du sulfate de streptomycine (100 pg/ml), du chlorhydrate de thiamine (4 pg/ml) et du sulfate de zinc 7H2O (0,0003 pg/ml), de préférence chauffés à 37 °C.

13. Échafaudage (21) à utiliser dans une préparation d'un modèle *in vitro* selon les revendications 1-6, comportant une lumière recouverte d'un endothélium fonctionnel et continu (21) comportant une monocouche cellulaire confluente, obtenu au moyen d'un procédé comprenant les étapes suivantes :
- préparer un échafaudage de forme tubulaire comportant les caractéristiques anatomiques et physiologiques dysfonctionnelles adaptées pour simuler une lésion, une déformation ou une détérioration due à un anévrisme, une sténose, des plaques de sclérose, des formes de tumeurs ou des cardiomyopathies, dans lequel l'échafaudage est fabriqué avec des déformations ou des défauts sur sa structure tubulaire, lesdites déformations ou défauts comprenant des courbures, des coudes, des constrictions, des dilatations, et est constitué de fibroïne de soie électrofilée, de copolymères d'acide polyglycolique/d'acide polylactique (PGA/PLA) ou de copolymères d'acide polyglycolique/polycaprolactone (PGA/PCL) ;
- ensemencer au moins une partie de la lumière intérieure dudit échafaudage avec des lignées cellulaires endothéliales de manière à obtenir une couche continue et homogène de cellules endothéliales ensemencées (procédé d'ensemencement), éventuellement ensemencer au moins une partie de la surface extérieure dudit échafaudage avec des lignées cellulaires musculaires ;
- favoriser la croissance desdites cellules endothéliales, et éventuellement desdites cellules musculaires, jusqu'à obtenir une couche continue et homogène de cellules endothéliales, pour obtenir ledit modèle *in vitro.*

14. Utilisation de l'échafaudage (21) selon l'une quelconque des revendications 1-6 ou 13, pour conduire des essais précliniques ou cliniques *in vitro* d'un médicament à usage humain destiné à être utilisé dans la région cardiovasculaire et vasculaire périphérique.
